# NEUE EUROPÄISCHE PATENTSCHRIFT

(11) **EP 2 066 611 B2**
(45) Veröffentlichungstag und Bekanntmachung des Hinweises auf die Entscheidung über den Einspruch: **27.02.2019**
(45) Hinweis auf die Patenterteilung: 22.06.2016
(21) Anmeldenummer: 07802810.7
(22) Anmeldetag: 22.08.2007
(51) Int. Cl.: C07C 51/43, C07C 51/377, C07C 57/04, C08F 120/06

(54) **VERFAHREN ZUR HERSTELLUNG VON DURCH KRISTALLISATION GEREINIGTER ACRYLSÄURE AUS HYDROXYPROPIONSÄURE SOWIE VORRICHTUNGEN DAZU**
PROCESS FOR PREPARING ACRYLIC ACID PURIFIED BY CRYSTALLIZATION FROM HYDROXYPROPIONIC ACID AND APPARATUS THEREFOR
PROCÉDÉ DE FABRICATION D'ACIDE ACRYLIQUE PURIFIÉ PAR CRISTALLISATION À PARTIR D'ACIDE HYDROXYPROPIONIQUE ET DISPOSITIFS ASSOCIÉS

(30) Priorität: 22.08.2006 DE 102006039203
(43) Veröffentlichungstag der Anmeldung: 10.06.2009
(73) Patentinhaber: Evonik Degussa GmbH, 45128 Essen (DE)
(72) Erfinder: KUPPINGER, Franz-Felix, 45768 Marl (DE); HENGSTERMANN, Axel, 48308 Senden (DE); STOCHNIOL, Guido, 45721 Haltern (DE); Bub, Prof. Dr., Günther, 45772 Marl (DE); Mosler, Dr., Jürgen, 45772 Marl (DE); Sabbagh, Dr., Andreas, 64625 Bensheim (DE)
(74) Vertreter: Lang, Arne
(86) Internationale Anmeldenummer: PCT/EP2007/058744
(87) Internationale Veröffentlichungsnummer: WO 2008/023039

(56) Entgegenhaltungen:
- EP-A1- 0 776 875
- EP-A1- 1 669 459
- WO-A-02/090312
- WO-A-2006/008083
- WO-A-2006/029821
- WO-A1-01/16346
- WO-A1-2005/073161
- WO-A2-02/42418
- WO-A2-03/062173
- WO-A2-03/082795
- DE-A1- 4 000 942
- DE-A1- 10 149 353
- US-A- 2 468 701
- US-A- 5 543 546
- US-A1- 2003 018 214
- <<<N O N - C I T E D D O C U M E N T>>> QATIBI et al.: "Anaerobic degradation of glycerol by Desulfovibrio fructosovorans and D. carbinolicus and evidence for glycerol-dependent utilization of 1,2-propanediol", Current Microbiology, vol. 36, 1998, pages 283-290, XP003027980,
- <<<N O N - C I T E D D O C U M E N T>>> SOBOLOV et al.: "METABOLISM OF GLYCEROL BY AN ACROLEIN-FORMING LACTO-BACILLUS", J. Bacteriol., vol. 79, 1960, pages 261-266, XP003027981,

## Beschreibung

Die vorliegende Erfindung betrifft ein Verfahren zur Herstellung von Acrylsäure, sowie eine Vorrichtung zur Herstellung von Acrylsäure.

Acrylsäure ist eine technisch sehr bedeutende Ausgangsverbindung. Sie dient unter anderem zur Herstellung von Polyacrylaten, insbesondere von vernetzten, teilneutralisierten Polyacrylaten, die im trockenen und im wesentlichen wasserfreien Zustand eine große Fähigkeit zur Wasserabsorption aufweisen. Diese kann mehr als das Zehnfache ihres Eigengewichts ausmachen. Aufgrund der hohen Absorptionsfähigkeit eignen sich absorbierende Polymere zur Einarbeitung in wasserabsorbierenden Strukturen und Gegenständen, wie z. B. Babywindeln, Inkontinenzprodukten oder Damenbinden. Diese absorbierenden Polymere werden in der Literatur auch als "Superabsorber" bezeichnet. In diesem Zusammenhang wird auf "Modern Superabsorbent Polymer Technology"; F.L. Buchholz, A.T. Graham, Wiley-VCH, 1998 verwiesen.

Üblicherweise wird Acrylsäure durch eine zweistufig verlaufende Gasphasenoxidation aus Propylen gewonnen, bei der zunächst Propylen unter Erhalt von Acrolein oxidiert wird, welches dann weiter zur Acrylsäure umgesetzt wird. Nachteilig bei diesem zweistufigen Verfahren zur Herstellung von Acrylsäure ist zum einen, dass die in beiden Reaktionsstufen angewandten Temperaturen, welche üblicherweise zwischen 300 und 450°C liegen, zur Bildung von unerwünschten Crackprodukten führen. Dieses wiederum hat zur Folge, dass eine unerwünscht hohe Menge an gegebenenfalls auch polymerisierbaren Verunreinigungen anfällt, die bei der Herstellung von Superabsorbern durch radikalische Polymerisation von Acrylsäure in Gegenwart von Vernetzern in das Polymerrückgrad eingebaut werden können. Diese wirkt sich nachteilig auf die Eigenschaften der Superabsorber aus. Weiterhin wirken insbesondere Aldehyde, wie etwa Furfural, Acrolein oder Benzaldehyd, als Inhibitoren bei der radikalischen Polymerisation, mit der Folge, dass die Polymere noch beachtliche Mengen an löslichen Bestandteilen beinhalten, sofern diese nicht aus der zur Polymerisation eingesetzten Acrylsäure durch aufwendige Reinigungsschritte entzogen werden.

Zu beachten ist auch, dass bei einem Einsatz von Superabsorbern in Hygieneartikeln und in Produkten zur Wundbehandlung die Anforderung an die toxische Unbedenklichkeit sehr hoch ist. Dieses führt dazu, dass die zur Herstellung der Superabsorber eingesetzten Edukte ebenso möglichst hohe Reinheiten besitzen müssen. Daher ist es von großer Bedeutung, Acrylsäure als ein Hauptedukt auf kostengünstige Weise so rein wie möglich zur Herstellung von Superabsorbern zur Verfügung zu stellen.

Ein weiterer Nachteil des herkömmlichen Verfahrens zur Herstellung von Acrylsäure besteht darin, dass das eingesetzte Edukt (Propylen) aus Erdöl und somit aus nicht-nachwachsenden Rohstoffen hergestellt wird, was vor allem im Hinblick auf die zunehmend schwerer und vor allem teurer werdende Erdölgewinnung aus ökonomischen Aspekten vor allem langfristig nachteilig ist.

Auch hier sind im Stand der Technik bereits einige Ansätze beschrieben, um diesem Problem zu begegnen. So ist insbesondere bekannt, Acrylsäure ausgehend von Hydroxypropionsäuren, beispielsweise aus 2-Hydroxypropionsäure oder 3-Hydroxypropionsäure, durch Dehydratisierung der Hydroxypropionsäure zu gewinnen.

Die Herstellung von 2-Hydroxypropionsäure ist unter anderem aus dem PEP Review 96-7 "Lactic acid by Fermentation" von Ronald Bray vom Juni 1998 auf fermentnativem Wege aus Biomasse wie Glucose oder Melasse und auf synthetischem Wege bekannt.

WO-A-03/62173 beschreibt die Herstellung von 3-Hydroxyprionsäure, welche unter anderem als Ausgangsprodukt für die Acrylsäuresynthese dienen kann. Dabei wird gemäß der Lehre der WO-A-03/62173 zunächst fermentativ aus Pyruvat α-Alanin gebildet wird, welches dann mittels des Enzyms 2,3-Aminomutase zu Beta-Alanin umgesetzt wird. Das β-Alanin wiederum wird über β-Alanyl-CoA, Acrylyl-CoA, 3-Hydroxypropionyl-CoA oder aber über Malonäuresemialdehyd zu 3-Hydroxypropionsäure umgesetzt, aus der nach einer Dehydratisierung Acrylsäure erhalten wird.

WO-A-02/42418 beschreibt einen weiteren Weg zur Herstellung von beispielsweise 3-Hydroxypropinsäure aus nachwachsenden Rohstoffen. Dabei wird Pyruvat zunächst zu Lactat umgesetzt, aus dem anschließend Lactyl-CoA gebildet wird. Das Lactyl-CoA wird dann über Acrylyl-CoA und 3-Hydroxypropionyl-CoA zu 3-Hydroxypropionsäure umgesetzt. Eine weitere, in der WO-A-02/42418 beschriebene Route zur Herstellung von 3-Hydroxypropionsäure sieht die Umsetzung von Glucose über Propionat, Propionyl-CoA, Acrylyl-CoA und 3-Hydroxypropionyl-CoA vor. Auch beschreibt diese Druckschrift die Umsetzung von Pyruvat zu 3-Hydroxypropionsäure über Acetyl-CoA und Malonyl-CoA. Die durch die jeweiligen Routen erhaltene 3-Hydroxypropionsäure kann durch Dehydratisierung zur Acrylsäure umgesetzt werden.

WO-A-01/16346 beschreibt die fermentative Herstellung von 3-Hydroxypropionsäure aus Glycerol, bei dem Mikroorganismen eingesetzt werden, welche das dhaB-Gen aus *Klebsiella pneumoniae* (ein Gen, welches für Glycerin-Dehydratase codiert) und ein Gen, welches für eine Aldehyd-Dehydrogenase codiert, exprimieren. Auf diese Weise wird aus Glycerin über 3-Hydroxypropionaldehyd 3-Hydroxypropionsäure gebildet, die dann durch Dehydratisierung in Acrylsäure überführt werden kann.

WO 2006/029821 offenbart eine Vorrichtung zur Herstellung von (Meth)acrylsäure, welche eine Syntheseeinheit, eine Derhydratisierungsstufe und auch eine Kristallisationseinheit beinhaltet.

WO 03/082795 offenbart die Herstellung von Acrylsäure aus einer Fermentationsbrühe enthaltend Hydroxy-Propionsäure. Dabei wird die Hydroxy-Propionsäure enthaltende Fermentationsbrühe zunächst einem Dehydrierungsverfahren unterworfen, um die Acrylsäure zu erhalten. Die Aufreinigung erfolgt mittels Destillation.

Unabhängig davon, über welchen enzymatischen Weg die Hydroxypropionsäure durch Fermentation erhalten wird, liegt im Anschluss an den fermentativen Prozess eine wässrige Zusammensetzung vor, welche neben der Hydroxypropionsäure noch zahlreiche Nebenprodukte beinhaltet, wie beispielsweise Zellen, nicht umgesetzte Biomasse, Salze und neben der Hydroxypropionsäure gebildete Stoffwechselprodukte.

Um die Hydroxypropionsäure als Ausgangsmaterial für die Herstellung von Acrylsäure zu verwenden ist es vorteilhaft, diese zunächst aufzukonzentrieren und aufzureinigen. Dabei ist im Zusammenhang mit der Aufreinigung von 3-Hydroxypropionsäure aus WO-A-02/090312 bekannt, der Fermentationslösung zunächst Ammoniak zur Neutralisation zuzusetzen, um die 3-Hydroxypropionsäure in ihr Ammoniumsalz zu überführen. Anschließend wird die so erhaltene Fermentationslösung mit einem hochsiedenden, organischen Extraktionsmittel in Kontakt gebracht und erhitzt, wobei Ammoniak und Wasser unter Vakuum abgezogen und die gebildete, freie 3-Hydroxypropionsäure in die organische Phase extrahiert wird. Auf diese Weise wird eine 3-Hydroxypropionsäure beinhaltende organische Phase erhalten, aus der die 3-Hydroxypropionsäure zurückextrahiert werden kann oder in der, nach Zusatz eines geeigneten Katalysators, die 3-Hydroxypropionsäure zu Acrylsäure umgesetzt werden kann. Der Nachteil des in der WO-A-02/090312 beschriebenen "Salt-Splitting"-Verfahrens besteht unter anderem darin, dass zum einen die aufzureinigende wässrige Phase die 3-Hydroxypropionsäure in einer Menge von mindestens 25 Gew.-% aufweisen muss, um eine effektive Aufreinigung durch das sogenannte "Salt-Splitting-Verfahren" zu ermöglichen. Da derart hohe 3-Hydroxypropionsäure-Konzentrationen in den derzeit bekannten Fermentationsprozessen zur Herstellung von 3-Hydroxypropionsäure nicht erzielt werden können, ist es erforderlich, die 3-Hydroxypropionsäure-Konzentration in der Fermentationslösung zunächst aufzukonzentrieren. Dieses geschieht üblicherweise durch Verdampfen des Wassers aus der Fermentationslösung. Weitere Nachteile des in der WO-A-02/090312 beschriebenen Aufreinigungsverfahrens bestehen darin, dass dieses Verfahren zum einen den Zusatz eines hochsiedenden organischen Lösungsmittels zur Fermentationslösung vorsieht, welches im weiteren Verfahrensverlauf von der 3-Hydroxypropionsäure abgetrennt werden muss, und dass zum anderen bei der Aufreinigung von 3-Hydroxypropionsäure aus Fermentationslösungen, welche zahlreiche organischen Nebenprodukte umfassen, auch diese organischen Nebenprodukte zumindest teilweise in das organische Lösungsmittel extrahiert werden. In diesem Fall ist es erforderlich, die beim Salt-Splitting erhaltene organische Phase weiter aufzureinigen, um 3-Hydroxypropionsäure mit einer zufrieden stellenden Reinheit zu erhalten.

Im Zusammenhang mit der Aufreinigung von 2-Hydroxypropionsäure (=Milchsäure) aus wässrigen Lösungen ist aus der WO-A-95/024496 bekannt, die wässrige Lösung zunächst mittels eines Extraktionsmittels umfassend ein mit Wasser nicht mischbares Trialkylamin mit insgesamt mindestens 18 Kohlenstoffen in Gegenwart von Kohlendioxid bei einem Partialdruck von mindestens 345 × 10³ Pascal unter Bildung einer wässrigen und einer organischen Phase kombiniert wird und anschließend die Milchsäure, welche sich in der organischen Phase befindet, beispielsweise mittels Wasser aus der organischen Phase extrahiert wird. Auch hier besteht der Nachteil des Aufreinigungsverfahrens darin, dass im Falle einer Fermentationslösung als Ausgangszusammensetzung nicht nur die Milchsäure, sondern auch andere Nebenprodukte in der organischen Phase gelöst werden, so dass bei der Rückextraktion mit Wasser keine reine Milchsäure-Lösung erhalten wird.

Bei der Dehydratisierung von Hydroxypropionsäure zu Acrylsäure fällt weiterhin Wasser an. Selbst bei dem Einsatz recht konzentrierter Hydroxypropionsäure-Lösungen oder gar purer Hydroxypropionsäure muss daher eine Aufreinigung eines wässrigen Gemisches aus Acrylsäure, Hydroxypropionsäure und ggf. bei der Dehydratisierung anfallender Nebenprodukte erfolgen.

WO-A-2004/76398 schlägt eine Vakuumdestillation mit Dodekanol als Zusatz zur Trennung eines Gemisches aus Acrylsäure und 3-Hydroxypropionsäre vor. Dieses schonende Verfahren ist jedoch wegen des Einsatzes des Dodekanols nachteilig.

Der vorliegenden Erfindung lag die Aufgabe zugrunde, die sich aus dem Stand der Technik ergebenden Nachteile im Zusammenhang zu lindern oder gar zu überwinden.

Insbesondere lag der vorliegenden Erfindung die Aufgabe zugrunde, ein Verfahren zur Herstellung von Acrylsäure aus einer fluiden Phase, vorzugsweise auf Basis einer wässrigen Fermentationslösung anzugeben, mit dem unter möglichst schonenden Bedingungen und einfach eine möglichst reine Acrylsäure bzw. eine möglichst reine wässrige Acrylsäure erhalten werden kann. Dieses Aufreinigungsverfahren sollte mit möglichst geringen oder gar ohne den Zusatz organischer Lösungsmittel durchgeführt werden können.

Des Weiteren lag der vorliegenden Erfindung die Aufgabe zugrunde, ein Verfahren zur Herstellung von Acrylsäure anzugeben, welches eine möglichst schonende und einfache Herstellung der Acrylsäure, insbesondere aus Biomasse, ermöglicht.

Auch lag der vorliegenden Erfindung die Aufgabe zugrunde, Vorrichtungen zur Herstellung von Acrylsäure bereitzustellen, mit denen solche Verfahren durchgeführt werden können.

Insgesamt soll das Ziel verfolgt werden, die jeweilige Verfahrensführung möglichst effizient und preiswert unter Erhalt möglichst reiner Endprodukte zu gestallten, wobei als Ausgangsstoffe möglichst regenerative Rohstoffe verwendet werden sollen. So soll ein Beitrag dazu geleistet werden, dass die auf nachwachsenden Rohstoffen basierende Herstellung von Acrylsäure nicht nur nachhaltiger und ökologisch vorteilhafter als die klassische petrochemische, meist über Propylen laufende Route zur Herstellung von Acrylsäure ist, sondern auch ökonomisch von Interesse ist.

Ein Beitrag zur Lösung der vorstehend genannten Aufgaben wird erfindungsgemäß geleistet durch das Verfahren zur Herstellung von Acrylsäure, durch die Vorrichtung zur Herstellung von Acrylsäure, wie in den jeweiligen Haupt- und Nebenansprüchen angegeben. Weitere Ausgestaltungen und Weiterbildungen, die jeweils einzeln oder beliebig miteinander kombiniert werden können, sind Gegenstand der jeweilig abhängigen Ansprüche.

Das erfindungsgemäße Verfahren zur Herstellung von Acrylsäure beinhaltet die Verfahrensschritte:
(a1) Bereitstellen einer Hydroxypropionsäure beinhaltenden wässrigen Phase P1,
(a2) Dehydratisieren der Hydroxypropionsäure unter Erhalt einer Acrylsäure beinhaltenden wässrigen Phase P2,
(a3) Aufreinigung der Acrylsäure beinhaltenden wässrigen Phase P2, durch eine Kristallisation unter Erhalt einer gereinigten Phase,
wobei die Dehydratisierung der Hydroxypropionsäure im Verfahrensschritt (a2) durch eine Flüssigphasendehydratisierung, mittels einer Reaktivdestillation erfolgt, wobei die Dehydratisierung in mindestens zwei Reaktoren durchgeführt wird, beinhaltend die folgenden Verfahrensschritte:
- Erhitzen der wässrige Phase P1 in mindestens einen ersten Reaktor R1 in Gegenwart eines homogenen oder heterogenen Katalysators unter Erhalt eines ersten, Acrylsäure beinhaltenden wässrigen Fluids F1_1 unter einem Druck Π1;
- Einbringen dieses Fluids Fl_1 in einen weiteren Reaktor R2;
- Erhitzen des in den Reaktor R2 eingebrachten Fluids F1_1 in Gegenwart eines Katalysators unter einem Druck Π2 unter Erhalt eines Fluides F1_2,
wobei Π2 und Π1 ungleich sind.

Die Begriffe "Acrylsäure", "Hydroxypropionsäure", "2-Hydroxypropionsäure" und "3-Hydroxypropionsäure", wie sie hierin verwendet werden, beschreiben dabei stets die entsprechende Carbonsäure in derjenigen Form, in der sie unter denen gegebenen pH-Bedingungen in den Phasen P1 bzw. P2 vorliegen. Die Begriffe umfassen somit stets die reine Säureform (Acrylsäure, Hydroxypropionsäure, 2-Hydroxypropionsäure bzw. 3-Hydroxypropionsäure), die reine Basenform (Acrylat, Hydroxypropionat, 2-Hydroxypropionat bzw. 3-Hydroxypropionat) sowie Mischungen aus protonierter und deprotonierter Form der Säuren.

Es ist in den erfindungsgemäßen Verfahren bevorzugt, dass mindestens ein, vorzugsweise mindestens zwei der Schritte des erfindungsgemäßen Verfahrens kontinuierlich erfolgen und nicht durch chargenweise Umsetzungen fortlaufend unterbrochen und wieder angefahren werden müssen. Bevorzugt erfolgen die mindestens die Schritte der Dehydratisierung und der Kristallisation und besonders bevorzugt alle Schritte kontinuierlich.

Die wässrige Phase P1 wird vorzugsweise erhalten durch ein Verfahren beinhaltend die Verfahrensschritte:
i) Herstellung, vorzugsweise fermentative Herstellung, von Hydroxypropionsäure aus einem biologischen Material, besonders bevorzugt aus Kohlenhydraten, insbesondere aus Glukose, oder aus Glycerin, in einer wässrigen Zusammensetzung unter Erhalt einer Hydroxypropionsäure sowie Mikroorganismen beinhaltenden, wässrigen Phase,
ii) gegebenenfalls Abtöten der Mikroorganismen, vorzugsweise durch Erhitzen dieser wässrigen Phase auf Temperaturen von mindestens 100°C, besonders bevorzugt mindestens 110°C und darüber hinaus bevorzugt mindestens 120°C für eine Dauer von mindestens 60 Sekunden, beispielsweise 10 Minuten und oder mindestens 30 Minuten,
iii) gegebenenfalls Abtrennen von Feststoffen, insbesondere von Mikroorganismen oder nicht umgesetztem biologischen Material, aus der wässrigen Phase, vorzugsweise mittels Sedimentation, Zentrifugation oder Filtration.

Zur vorzugsweise fermentativen Herstellung der Hydroxypropionsäure aus einem biologischen Material im Verfahrensschritt i) werden vorzugsweise rekombinante Mikroorganismen, besonders bevorzugt rekombinante Bakterien-, Pilz- oder Hefezellen eingesetzt. Erfindungsgemäß besonders bevorzugte rekombinante Mikroorganismen sind Bakterien der Gattungen *Corynebacterium, Brevibacterium, Bacillus, Lactobacillus, Lactococcus, Candida, Pichia, Kluveromyces, Saccharomyces, Bacillus, Escherichia* und *Clostridium,* wobei *Bacillus flavum, Bacillus lactofermentum, Escherichia coli, Saccharomyces cerevisiae, Kluveromyces lactis, Candida blankii, Candida rugosa, Corynebacterium glutamicum, Corynebacterium efficiens* und *Pichia postoris* darüber hinaus bevorzugt und *Corynebacterium glutamicum* am meisten bevorzugt ist bzw. sind.

Dabei ist es erfindungsgemäß weiterhin bevorzugt, dass die Mikroorganismen derart gentechnisch verändert sind, dass sie im Vergleich zu ihrem Wildtyp eine gesteigerte, vorzugsweise eine um einen Faktor von mindestens 2, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Bildung von Hydroxypropionsäure aus einem biologischen Material, vorzugsweise aus Kohlenhydraten wie Glukose oder aus Glycerin, aufweisen. Diese Steigerung der Hydroxypropionsäure-Bildung kann dabei grundsätzlich über alle dem Fachmann bekannten Stoffwechselwege, im Falle der Bildung von 3-Hydroxypropionsäure insbesondere über die in den Druckschriften WO-A-03/62173, WO-A-02/42418 und WO-A-01/16346 beschriebenen Routen und im Falle der Bildung von 2-Hydroxypropionsäure insbesondere aus Bakterienstämmen der Gattung *Bacillus* oder *Lactobacillus,* beispielsweise auf der in der DE 40 00 942 C2 beschrieben Art und Weise, erfolgen. Besonders bevorzugt erfolgt die Bildung von Hydroxypropionsäuren, insbesondere von 2- oder 3-Hydroxypropionsäuren, mittels rekombinanter Mikroorganismen, in denen die Aktivität eines oder mehrer für die Bildung der entsprechenden Hydroxypropionsäuren relevanten Enzyme erhöht worden ist, wobei die Erhöhung der Enzymaktivitäten durch dem Fachmann bekannte Maßnahmen, insbesondere durch Mutation oder Erhöhung der Genexpression erfolgen kann.

Die gentechnisch veränderten Mikroorganismen können kontinuierlich oder diskontinuierlich im batch-Verfahren (Satzkultivierung) oder im *fed-batch*-Verfahren (Zulaufverfahren) oder *repeated-fed-batch*-Verfahren (repetitives Zulaufverfahren) zum Zwecke der Produktion von Hydroxypropionsäure mit einem geeigneten Nährmedium in Kontakt und somit kultiviert werden. Denkbar ist auch ein semikontinuierliches Verfahren, wie es in der GB-A-1009370 beschrieben wird. Eine Zusammenfassung über bekannte Kultivierungsmethoden sind im Lehrbuch von Chmiel ("Bioprozesstechnik 1. Einführung in die Bioverfahrenstechnik" (Gustav Fischer Verlag, Stuttgart, 1991)) oder im Lehrbuch von Storhas ("Bioreaktoren und periphere Einrichtungen", Vieweg Verlag, Braunschweig/Wiesbaden, 1994) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D. C., USA, 1981) enthalten.

Als biologisches Material, insbesondere als Kohlenstoffquelle, können Zucker und Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Besonders bevorzugt ist der Einsatz von Kohlehydraten, insbesondere Monosaccharide, Oligosaccharide oder Polysaccharide, wie dies in US 6,01,494 und US 6,136,576 beschrieben ist, von C5-Zuckern oder von Glycerin.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Natriumbicarbonat, Kaliumhydroxid, Ammoniak bzw. Ammoniakwasser oder saure Verbindungen wie Phosphorsäure oder Schwefelsäure in geeigneter Weise eingesetzt, wobei der Zusatz von Ammoniak besonders bevorzugt ist. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoff-haltige Gasmischungen wie z. B. Luft in die Kultur eingetragen. Die Temperatur der Kultur liegt normalerweise bei 20°C bis 45°C und vorzugsweise bei 25°C bis 40°C.

Im Zusammenhang mit dieser besonderen Ausführungsform des erfindungsgemäßen Verfahrens ist es bevorzugt, dass die im Verfahrensschritten (a1) hergestellte wässrige Phase P1 eine Zusammensetzung Z1 aufweist, beinhaltend
(Z1_1) 1 bis 40 Gew.-%, vorzugsweise 5 bis 30 Gew.-% und am meisten bevorzugt 10 bis 20 Gew.-% Hydroxypropionsäure, vorzugsweise 2- oder 3-Hydroxypropionsäure und besonders bevorzugt 3-Hydroxypropionsäure, Salze dieser Säuren oder Mischungen davon,
(Z1_2) 0,1 bis 5 Gew.-%, vorzugsweise 0,3 bis 2,5 Gew.-% und am meisten bevorzugt 0,5 bis 1 Gew.-% anorganische Salze,
(Z1_3) 0,1 bis 30 Gew.-%, vorzugsweise 0,5 bis 20 Gew.-% und am meisten bevorzugt 1 bis 10 Gew.-% von der Hydroxypropionsäure verschiedene, organische Verbindungen,
(Z1_4) 0 bis 50 Gew.-%, vorzugsweise 1 bis 40 Gew.-%, vorweise 5 bis 20 Gew.-% und am meisten bevorzugt 1 bis 10 Gew.-% Feststoffe, insbesondere Feststoffe von feinen Pflanzenteilen bzw. Zellen und/oder Zellfragmenten, insbesondere von Mikroorganismen oder nicht umgesetztem biologischen Material, sowie
(Z1_5) 20 bis 90 Gew.-% ,vorzugsweise 30 bis 80 Gew.-% und am meisten bevorzugt 40 bis 70 Gew.-% Wasser,
wobei die Summe der Komponenten (Z1_1) bis (Z1_5) 100 Gew.-% beträgt.

Diese fluide Phase weist vorzugsweise einen pH-Wert in einem Bereich von 5 bis 8, vorzugsweise 5,2 bis 7 und besonders bevorzugt 5,5 bis 6,5 auf.

Bei dem Salz der Hydroxypropionsäure handelt es sich vorzugsweise um das Natriumsalz, das Kaliumsalz, das Kalziumsalt, das Ammoniumsalz oder Mischungen hieraus, wobei das Ammoniumsalz besonders bevorzugt ist.

Die anorganischen Salze sind vorzugsweise ausgewählt aus der Gruppe beinhaltend Natriumchlorid, Kaliumchlorid, Phosphate wie Natriumphosphat, Natriumcarbonat, Natriumhydrogencarbonat, Natriumdihydrogenphosphat oder Dinatriumhydrogenphosphat. Magnesiumsulfat, Eisensulfat, Kalziumchlorid, Kalziumsulfat oder Ammoniumsalze wie Ammoniumsulfat, Ammoniumchlorid, Ammoniumphosphat, Ammoniumcarbonat oder Ammoniumnitrat.

Die von der Hydroxypropionsäure verschiedenen organischen Verbindungen beinhaltend unter anderem nicht umgesetztes biologisches Material, wie beispielsweise Kohlenhydrate oder Glycerin, Stoffwechselprodukte wie beispielsweise Lactat, Pyruvat oder Ethanol, Antibiotika, Antischaummittel, organische Puffersubstanzen wie etwa HEPES, Aminosäuren, Vitamine, Peptone, Harnstoff oder Stickstoffhaltige-Verbindungen, wie sie beispielsweise in Hefeextrakt, Fleischextrakt, Malzextrakt, Maisquellwasser und Sojabohnenmehl enthalten sind.

Die im Anschluss an die Fermentation erhaltene Hydroxypropionsäure und Mikroorganismen beinhaltende wässrige Phase P1 (=Fermentationslösung) kann in einem weiteren Verfahrensschritt ii) thermisch behandelt werden, um die noch enthaltenen Mikroorganismen abzutöten. Dieses geschieht vorzugsweise dadurch, dass diese wässrige Phase P1 auf Temperaturen von mindestens 100°C, besonders bevorzugt mindestens 110°C und darüber hinaus bevorzugt mindestens 120°C für eine Dauer von mindestens 60 Sekunden, vorzugsweise mindestens 10 Minuten und darüber hinaus bevorzugt mindestens 30 Minuten erhitzt wird, wobei das Erhitzen vorzugsweise in dem Fachmann dafür bekannten Vorrichtungen, wie etwa einem Autoklaven, erfolgt. Denkbar ist auch die Abtötung der Mikroorganismen durch energiereiche Strahlung, wie beispielsweise UV-Strahlung, wobei ein Abtöten der Mikroorganismen durch Erhitzen besonders bevorzugt ist.

Weiterhin kann es bei dieser besonderen Ausführungsform des erfindungsgemäßen Verfahrens bevorzugt sein, dass vor, während oder nach dem Abtöten der Mikroorganismen im Verfahrensschritt ii) Feststoffe, insbesondere feine Pflanzenteile bzw. Zellen und/oder Zellfragmente, insbesondere Mikroorganismen oder nicht umgesetztes biologisches Material in einem weiteren Verfahrensschritt iii) von der wässrigen Phase P1, die im Anschluss an die Fermentation erhalten wird, abgetrennt werden, wobei dieses Abtrennen durch alle dem Fachmann bekannten Verfahren zur Abtrennung von Feststoffen aus flüssigen Zusammensetzungen erfolgen kann, vorzugsweise jedoch durch Sedimentation, Zentrifugation oder Filtration, wobei ein Abtrennen durch Filtration am meisten bevorzugt ist. Dabei können alle Filtrationsverfahren zur Anwendung gelangen, die im Kapitel 4.1.3 "Filtrieren und Auspressen" in "Grundoperationen Chemischer Verfahrenstechnik", Wilhelm R. A. Vauck und Hermann A. Müller, WILEY-VCH-Verlag, 11. überarbeitete und erweiterte Auflage, 2000, genannt und dem Fachmann für die Abtrennung von Feststoffen, insbesondere von Mikroorganismen aus Fermentationslösungen geeignet erscheinen.

Bevor die auf diese Weise erhaltene, gegebenenfalls von Feststoffen befreite Fermentationslösung als wässrige Phase P1 dem Verfahrensschritt (a2) unterzogen wird, kann es auch sinnvoll sein, den Wassergehalt dieser wässrigen Phase mittels Verdampfung oder Destillation, Umkehrosmose, Elektroosmose, azeotrope Destillation, Elektrodialyse oder Mehrphasentrennung zu reduzieren, insbesondere um einen Faktor von kleiner als 0,8, insbesondere kleiner als 0,5, beispielsweise kleiner als 0,3. Dabei bedeutet eine Reduktion um einen Faktor von kleiner als 0,8, dass der Wassergehalt der Fermentationslösung nach dem Reduzieren weniger als 80 % des Wassergehaltes der Fermentationslösung vor dem Reduzieren beträgt.

Insbesondere ist es erfindungsgemäß bevorzugt, den Wassergehalt so weit zu reduzieren, dass die Konzentration an Hydroxypropionsäure oder an deren Salzen in der Fermentationslösung mindestens 10 Gew.-%, besonders bevorzugt mindestens 20 Gew.-%, darüber hinaus bevorzugt mindestens 30 Gew.-% und am meisten bevorzugt mindestens 40 Gew.-% beträgt.

In einer Abwandlung der Erfindung beinhaltet das Verfahren zur Herstellung der wässrigen Phase P1 weiterhin den Verfahrensschritt (iv), in dem zumindest ein Teil des Wassers in der Fermentationslösung abgetrennt wird, wobei diese Abtrennung vorzugsweise durch die Bildung einer ersten Phase und einer zweiten Phase aus der wässrigen Phase P1, erfolgt. Weiterhin ist es bevorzugt, dass diese Bildung der ersten und zweiten Phase durch mindestens eines oder auch mindestens zwei oder mehr der folgenden Trennverfahren erfolgt:
- Verdampfung oder Destillation,
- Umkehrosmose,
- Elektroosmose,
- azeotrope Destillation,
- Elektrodialyse,
- Mehrphasentrennung,
wobei die erste und die zweite Phase verschiedene Konzentrationen an Acrylsäure aufweisen und im Anschluss an die Bildung dieser beiden Phasen die Phasen unter Erhalt einer Acrylsäure beinhaltenden gereinigten Phase voneinander getrennt werden.

Grundsätzlich kann der Verfahrensschritt (iv) vor dem Verfahrensschritt (a2) oder aber, wie etwa bei der nachfolgend beschrieben Reaktivdestillation, zeitgleich mit dem Verfahrensschritt (a2) durchgeführt wird. Durch den Verfahrensschritt (iv) wird aus der Phase P1 eine vorgereinigte erste bzw. zweite Phase gebildet, die denn der Dehydratisierungsreaktion im Verfahrensschritt (a2) unterworfen wird.

Als Verdampfungsvorrichtung können alle im Kapitel 10.1 "Verdampfen" in "Grundoperationen Chemischer Verfahrenstechnik", Wilhelm R. A. Vauck und Hermann A. Müller, WILEY-VCH-Verlag, 11. überarbeitete und erweiterte Auflage, 2000, genannten Vorrichtung eingesetzt werden, die dem Fachmann für eine Verdampfung von Wasser aus einer von Feststoffen befreiten Fermentationslösungen geeignet erscheinen.

Die Umkehrosmose - auch Reverseosmose oder Hyperfiltration genannt - ist eine Druckfiltration an einer semipermeablen Membran, bei der insbesondere ein Druck benutzt wird, um den natürlichen Osmose-Prozess umzukehren. Der anzuwendende Druck muss dabei größer sein als der Druck, der durch das osmotische Verlangen zum Konzentrationsausgleich entstehen würde. Die osmotische Membran, die nur die Trägerflüssigkeit (Solvent) durchlässt und die gelösten Stoffe (Solute) zurückhält, muss derartig ausgelegt sein, diesen oft hohen Druck standzuhalten. Wenn der Druckunterschied das osmotische Gefälle mehr als ausgleicht, passen die Solventmoleküle wie bei einem Filter durch die Membran, während die Verunreinigungsmoleküle zurückgehalten werden. Der osmotische Druck steigt mit zunehmendem Konzentrationsunterschied und kommt zum Stillstand, wenn der natürliche osmotische Druck gleich dem vorgegebenen Druck ist. Bei einer kontinuierlichen Verfahrensweise wird das Konzentrat stetig abgeführt. Es können Durchflussmembranen verwendet werden. Die Phase P1 wird dabei unter Drücken von in der Regel 2 bis 15 MPa gegen eine Porenmembran, vorzugsweise aus Celluloseacetat oder Polyamid, gepresst, wobei Wasser, nicht aber die Hydroxypropionsäure oder deren Salz durch die Poren der Membran hindurchtreten können. Geeignete Membranmaterialien und Vorrichtungen können unter anderem dem Kapitel 10.7 "Permeation" in "Grundoperationen Chemischer Verfahrenstechnik", Wilhelm R. A. Vauck und Hermann A. Müller, WILEY-VCH-Verlag, 11. überarbeitete und erweiterte Auflage, 2000, entnommen werden.

Bei einer Elektroosmose stellt sich in einem Gleichgewichtszustand der elektroosmotische Druck ein, mit dem ebenso eine Trennung der Hydroxypropionsäure von Verunreinigung wie z. B. Wasser getrennt werden kann. Bei der Elektrosomose wird ebenfalls Wasser über eine semipermeable Membran aus einer wässrigen Zusammensetzung selektiv abgetrennt, wobei jedoch im Gegensatz zur Umkehrosmose das Wasser nicht über einen Überdruck, sondern durch das Anlegen einer elektrischen Spannung von in der Regel 6 bis 20 V durch die semipermeable Membran getrieben wird.

Bei der Azeotropendestillation bzw. Azeotropenrektifikation wird durch Zusatz einer dritten Komponente ein azeotropes Gemisch getrennt. Die Rektifikation ist ein thermisches Trennverfahren und stellt eine Weiterentwicklung der Destillation oder eine Hintereinanderschaltung vieler Destillationsschritte dar. Die wesentlichen Vorteile der Rektifikation sind, dass die Anlage kontinuierlich betrieben werden kann und das der Trenneffekt im Vergleich zur Destillation um ein Vielfaches höher ist, da der Dampf mit der Flüssigkeit in Kontakt steht. Daraus resultiert, dass der schwerer siedende Anteil kondensiert und durch die freiwerdende Kondensationswärme leichter flüchtige Bestandteile der Flüssigkeitsphase verdampfen. Die Kontaktfläche zwischen der Dampf- und Flüssigphase wird durch Einbauten (z. B. Glockenböden) bereitgestellt. Wie auch bei der Destillation können unter normalen Bedingungen nur nicht azeotrope Gemische getrennt werden. Ist es erforderlich ein azeotropes Gemisch zu trennen, wird der azeotrope Punkt verschoben und darf nicht im Konzentration- und Temperaturbereich der Anlage liegen. Eine Verschiebung erfolgt z. B. durch Betriebsdruckänderung oder durch Zugabe eines bestimmten Hilfsstoffes. Die azeotrope Destillation erfolgt insbesondere unter Verwendung von organischen Lösungsmitteln wie z. B. Toluol oder Dodekanol als Schlepper, welches mit Wasser ein Azeotrop bildet. Anschließend wird die mit dem organischen Lösungsmittel in Kontakt gebrachte wässrige Lösung destilliert, wobei Wasser und das organische Lösungsmittel aus der Zusammensetzung abgetrennt werden.

Unter einer Elektrodialyse versteht man die Aufspaltung einer chemischen Verbindung unter der Einwirkung des elektrischen Stroms. Bei der Elektrodialyse werden zwei Elektroden in die wässrige Phase getaucht, wobei es an der Kathode zur Bildung von Wasserstoff und an der Anode zur Bildung von Sauerstoff kommt. Auf diese Weise entstehen ebenfalls zwei Phasen, nämlich die um zumindest einen Teil des Wassers befreite wässrige Phase und die aus Wasserstoff und Sauerstoff bestehende Gasphase. Hierbei wird insbesondere eine der fluiden Phase enthaltende Komponente (z. B. Wasser), in seine Bestandteile überführt, die als Gas oder (z.B. mittels einer nach der elektrolytischen Reaktion bewirkten Fällungsreaktion oder einer Feststoffanreicherung an einer Elektrode) als Feststoff entzogen werden können.

Bei der Mehrphasentrennung, wie sie beispielsweise in der WO-A-02/090312 beschrieben ist, wird die wässrige Phase P1, in der vorzugsweise die Hydroxypropionsäure durch den Zusatz von Ammoniak zumindest teilweise in das Ammoniumsalz überführt worden ist, in einem ersten Verfahrensschritt mit einem hochsiedenden, vorzugsweise nicht mit Wasser mischbaren, organischen Lösungsmittel bzw. mit einem hochsiedenden Lösungsmittelgemisch, in Kontakt gebracht. Anschließend wird die mit dem hochsiedenden, vorzugsweise nicht mit Wasser mischbaren, organischen Lösungsmittel bzw. mit einem hochsiedenden Lösungsmittelgemisch in Kontakt gebrachten Zusammensetzung auf eine Temperatur von vorzugsweise 20 bis 200°C, besonders bevorzugt 40 bis 120°C erhitzt, wobei Ammoniak und Wasserdampf aus der Zusammensetzung entweichen und die Hydroxypropionsäure in der Säureform in das hochsiedende organische Lösungsmittel bzw. in das hochsiedende Lösungsmittelgemisch extrahiert und eine Hydroxypropionsäure beinhaltende organische Phase erhalten wird. Dieses auch als "Salt-Splitting" bezeichnete Verfahren ist in der WO-A-02/090312 beschrieben. Die auf diese Weise erhaltene organische Phase kann als Phase P1 im Verfahrensschritt (a2) eingesetzt werden.

Als bevorzugte organische Lösungsmittel werden dabei diejenigen Lösungsmittel eingesetzt, die in der WO-A-02/090312 als bevorzugte Extraktionsmittel genannt werden. Bevorzugte hochsiedende organische Lösungsmittel sind dabei organische Amine, insbesondere Trialkylamine mit einer Gesamtanzahl an Kohlenstoffatomen von mindestens 18 und einem Siedepunkt von mindestens 100°C, vorzugsweise mindestens 175°C bei Atmosphärendruck, wie Trioctylamin, Tridecylamin oder Tridodecylamin sowie Lösungsmittemischungen aus diesen Trialkylaminen und hochsiedenden Kohlenstoff-Sauerstoff-Verbindungen wie beispielsweise Alkoholen, hochsiedenden Phosphor-Sauerstoff-Verbindungen wie Phosphorsäureestern, hochsiedenden Phosphinsulfiden oder hochsiedenden Alkylsulfiden, wobei "hochsiedend" vorzugsweise bedeutet, dass diese Verbindungen einen Siedepunkt von mindestens 175°C bei Atmosphäredruck aufweisen.

Nachdem die wässrige Phase P1, beinhaltend das Ammoniumsalz der Hydroxypropionsäure mit diesen Lösungsmitteln oder Lösungsmittelgemischen gemäß der in der WO-A-02/090312 beschriebenen Vorgehensweise in Kontakt gebracht worden ist, wird die so erhaltene Zusammensetzung erhitzt, wobei Wasserdampf und Ammoniak entweichen. Gegebenfalls kann durch einen Unterdruck die Freisetzung dieser unter den Temperaturbedingungen zumindest teilweise gasförmige vorliegenden Komponenten gefördert werden. Schließlich wird eine organische Phase erhalten, welche die Hydroxypropionsäure in ihrer Säureform beinhaltet und die dann als Phase P1 im Verfahrensschritt (a2) eingesetzt werden kann. Denkbar ist auch, die Hydroxypropionsäure mit Wasser zurückzuextrahieren und die so erhaltene wässrige Hydroxypropionsäure-Lösung dem Verfahrensschritt (a2) zu unterziehen.

Denkbar ist weiterhin eine Mehrphasentrennung, wie sie beispielsweise in der WO-A-95/024496 beschrieben ist. Gemäß diesem Trennverfahren wird eine Hydroxycarbonsäure-Lösung, in der die Hydroxycarbonsäure durch den Zusatz basischer Verbindungen, wie etwa Natriumbicarbonat, als Hydroxypropionat vorliegt, gegebenenfalls nach einer Filtration und nach einem Abdampfen des Wasser, mittels eines Extraktionsmittels umfassend ein mit Wasser nicht mischbares Trialkylamin mit insgesamt mindestens 18 Kohlenstoffen in Gegenwart von Kohlendioxid bei einem Partialdruck von mindestens 345 × 10³ Pascal unter Bildung einer wässrigen und einer organischen Phase kombiniert und anschließend die Hydroxypropionsäure, welche sich in der organischen Phase befindet, beispielsweise mittels Wasser aus der organischen Phase extrahiert. Auch bei diesem Mehrphasentrennverfahren kann sowohl die Hydroxypropionsäure enthaltene organische Phase als auch die nach einer Rückextraktion mit Wasser erhaltene, Hydroxypropionsäure enthaltene wässrige Phase dem Verfahrensschritt (a2) unterzogen werden.

Das Dehydratisieren der Hydroxypropionsäure und damit die Synthese der Acrylsäure im Verfahrensschritt (a2) erfolgt vorzugsweise durch Erhitzen der gegebenenfalls von Mikroorganismen befreiten und gegebenenfalls entwässerten wässrigen Fermentationslösung oder aber der bei Anwendung der Mehrphasentrennung erhaltenen organischen oder wässrigen Phase, wobei dieses Erhitzen besonders bevorzugt in Gegenwart eines Katalysators erfolgt.

Als Dehydratisierungskatalysatoren kommen sowohl saure als auch alkalische Katalysatoren in Betracht. Saure Katalysatoren sind insbesondere wegen der geringen Neigung zur Oligomerenbildung bevorzugt. Der Dehydratisierungskatalysator kann sowohl als homogener als auch als heterogener Katalysator eingesetzt werden. Wenn der Dehydratisierungskatalysator als heterogener Katalysator vorliegt, ist es bevorzugt, dass der Dehydratisierungskatalysator mit einem Träger x. in Kontakt steht. Als Träger x. kommen alle dem Fachmann als geeignet erscheinenden Feststoffe in Betracht. In diesem Zusammenhang ist es bevorzugt, dass diese Feststoffe geeignete Porenvolumina aufweisen, die zur guten Anbindung und Aufnahme des Dehydratisierungskatalysators geeignet sind. Außerdem sind Gesamtporenvolumen nach DIN 66133 in einem Bereich von 0,01 bis 3 ml/g bevorzugt und in einem Bereich von 0,1 bis 1,5 ml/g besonders bevorzugt. Zudem ist es bevorzugt, dass die als Träger x. geeigneten Feststoffe eine Oberfläche im Bereich von 0,001 bis 1000 m²/g, vorzugsweise im Bereich von 0,005 bis 450 m²/g und darüber hinaus bevorzugt im Bereich von 0,01 bis 300 m²/g nach BET-Test gemäß DIN 66131 aufweisen. Als Träger für den Dehydratisierungskatalysator kann zum einen ein Schüttgut, das einen mittleren Teilchendurchmesser im Bereich von 0,1 bis 40 mm, vorzugsweise im Bereich von 1 bis 10 mm und darüber hinaus bevorzugt im Bereich von 1,5 bis 5 mm aufweist, eingesetzt werden. Ferner kann die Wand des Dehydratisierungsreaktors als Träger dienen. Weiterhin kann der Träger an sich sauer oder basisch sein oder ein saurer oder basischer Dehydratisierungskatalysator auf einen inerten Träger aufgebracht werden. Als Aufbringtechniken sind insbesondere Eintauchen bzw. Imprägnieren oder das Einarbeiten in eine Trägermatrix zu nennen.

Als Träger x., die auch Dehydratisierungskatalysatoreigenschaften aufweisen können, eignen sich insbesondere natürliche oder synthetische silikatische Stoffe, wie insbesondere Mordenit, Montmorillonit, saure Zeolithe, saure Aluminiumoxide, γ-Al₂O₃, mit ein-, zwei oder mehrbasigen anorganischen Säuren, insbesondere Phosphorsäure, oder saure Salze anorganischer Säuren belegte Trägerstoffe, wie oxidische oder silikatische Stoffe, beispielsweise Al₂O₃, TiO₂; Oxide und Mischoxide, wie beispielsweise Gamma-Al₂O₃ und ZnO-Al₂O₃- Mischoxide der Heteropolysäuren.

Es entspricht einer erfindungsgemäßen Ausführungsform, dass der Träger x. mindestens teilweise aus einer oxidischen Verbindung besteht. Derartige oxidische Verbindungen sollten mindestens eines der Elemente aus Si, Ti, Zr, Al, P oder eine Kombination von mindestens zwei davon aufweisen. Derartige Träger können auch selber durch ihre sauren bzw. basischen Eigenschaften als Dehydratisierungskatalysator wirken. Eine bevorzugte sowohl als Träger als x. als auch als Dehydratisierungskatalysator wirkende Verbindungsklasse beinhalten Silizium-Aluminium-Phosphoroxide. Bevorzugte basische sowohl als Dehydratisierungskatalysator als auch als Träger x. fungierende Stoffe beinhalten Alkali, Erdalkali, Lanthan, Lanthanoide oder eine Kombination von mindestens zwei davon in ihrer oxidischen Form, wie beispielsweise Li₂O-, Na₂O-, K₂O- Cs₂O-, MgO-, CaO-, SrO- oder BaO- oder La₂O₃-haltige Stoffe. Derartige saure oder basische Dehydratisierungskatalysatoren sind sowohl bei der Degussa AG als auch bei der Südchemie AG kommerziell erhältlich. Eine weitere Klasse stellen Ionenaustauscher dar. Auch diese können sowohl in basischer als auch in saurer Form vorliegen.

Als homogene Dehydratisierungskatalysatoren kommen insbesondere anorganische Säuren, vorzugsweise Phosphor beinhaltende Säuren und darüber hinaus bevorzugt Phosphorsäure in Betracht. Diese anorganischen Säuren können auf dem Träger x. durch Eintauchen bzw. Imprägnieren immobilisiert werden.

Insbesondere bei der Gasphasendehydratisierung hat sich der Einsatz von heterogenen Katalysatoren besonders bewährt. Bei der Flüssigphasendehydratisierung werden jedoch sowohl homogene als auch heterogene Dehydratisierungskatalysatoren eingesetzt.

Zudem ist es bevorzugt, dass in dem erfindungsgemäßen Verfahren zur Herstellung von Acrylsäure ein Dehydratisierungskatalysator mit einem Ho-Wert im Bereich von +1 bis -10, vorzugsweise in einem Bereich von +2 bis -8,2 und darüber hinaus bevorzugt bei der Flüssigphasendehydratisierung in einem Bereich von +2 bis -3 und in der Gasphasendehydratisierung in einem Bereich von -3 bis -8,2 eingesetzt wird. Der Ho-Wert entspricht der Säurefunktion nach Hammett und lässt sich durch die sogenannte Amintritation und Verwendung von Indikatoren oder durch Absorption einer gasförmigen Base ermitteln - siehe "Studies in Surface Science and Catalytics", vol. 51, 1989: "New solid Acids and Bases, their catalytic Properties", K. Tannabe et al. Weitere Einzelheiten zur Herstellung von Acrolein aus Glycerin sind weiterhin DE 42 38 493 C1 zu entnehmen.

Gemäß einer besonderen Ausführungsform des erfindungsgemäßen Verfahrens zur Herstellung von Acrylsäure wird als saurer Feststoffkatalysator ein mit einer anorganischen Säure, vorzugsweise mit Phosphorsäure oder mit Supersäuren wie etwa sulfatisiertem oder phosphatisiertem Zirkoniumoxid in Kontakt gebrachter porenförmiger Trägerkörper eingesetzt, der vorzugsweise zu mindestens 90 Gew.-%, darüber hinaus bevorzugt zu mindestens 95 Gew.-% und am meisten bevorzugt zu mindestens 99 Gew.-% auf einem Siliziumoxid, vorzugsweise auf SiO₂, basiert. Das in Kontakt bringen des porenförmigen Trägerkörpers mit der anorganischen Säure erfolgt vorzugsweise durch das Imprägnieren des Trägerkörpers mit der Säure, wobei diese vorzugsweise in einer Menge in einem Bereich von 10 bis 70 Gew.-%, besonders bevorzugt in einem Bereich 20 bis 60 Gew.-% und darüber hinaus bevorzugt in einem Bereich von 30 bis 50 Gew.-%, bezogen auf das Gewicht des Trägerkörpers, mit diesem in Kontakt gebracht und anschließend getrocknet wird. Im Anschluss an die Trocknung wird der Trägerkörper zur Fixierung der anorganischen Säure erhitzt, vorzugsweise auf eine Temperatur in einem Bereich von 300 bis 600 °C, darüber hinaus bevorzugt in einem Bereich von 400 bis 500 °C.

Die Dehydratisierung der Hydroxypropionsäure im Verfahrensschritt (a2) erfolgt durch eine Flüssigphasendehydratisierung mittels einer sogenannten "Reaktivdestillation".

"Reaktivdestillation" ist eine Reaktion, aus der eine Komponente durch Verdampfen abgetrennt wird und dadurch das chemische Gleichgewicht dieser Reaktion beeinflusst wird. Insbesondere bezeichnet man als "Reaktivdestillation" die simultane Durchführung einer chemischen Reaktion und einer Destillation in einer Kolonne, insbesondere einer Gegenstromkolonne. Diese simultane Durchführung von Reaktion und Stofftrennung ist besonders vorteilhaft bei solchen Reaktionen, bei denen aufgrund der Lage des chemischen Gleichgewichtes die Edukte nicht vollständig in die gewünschten Produkte umgesetzt werden. Durch die simultane Abtrennung der Reaktionsprodukte aus dem Reaktionsraum gelingt auch in diesen Fällen eine fast vollständige Umsetzung in einem einzigen Apparat. Weitere Vorteile der Reaktivdestillation sind die Unterdrückung unerwünschter Nebenreaktionen, die thermische Nutzung einer exothermen Reaktionswärme für die Destillation und die Erleichterung der nachfolgenden Produktaufbereitung. Im Zusammenhang mit der Dehydratisierung von Hydroxypropionsäure beschreibt der Begriff "Reaktivdestillation" demnach vorzugsweise ein Verfahren, bei dem die die Hydroxypropionsäure beinhaltende wässrige Phase P1, welche im Verfahrensschritt (a1) erhalten wurde, gegebenenfalls nach einer Weiterbehandlung mittels eines der oder aller Verfahrensschritte (i) bis (iv), in Gegenwart eines die Dehydratisierung fördernden Katalysators unter Bedingungen erhitzt wird, unter denen die Hydroxypropionsäure zumindest teilweise zur Acrylsäure umgesetzt wird und unter denen zeitgleich Wasser, welches sich in der Reaktionslösung befindet, abdestilliert werden kann. Auf diese Weise wird zeitgleich die Hydroxypropionsäure zur Acrylsäure dehydratisiert und die Reaktionsmischung destilliert, wobei Wasser als Destillat abgetrennt wird und ein Acrylsäure-haltiges Sumpfprodukt erhalten wird.

Um Decarboxylierungsreaktionen während der Reaktivdestillation zu vermeiden, ist es weiterhin besonders vorteilhaft, diese Reaktivdestillation in einer CO₂-Atmosphäre durchzuführen. Dieses hat zudem den Vorteil, dass das CO₂ zumindest teilweise in Form von Kohlensäure in der wässrigen Reaktionsmischung vorliegt und als Säure zugleich als Katalysator die Dehydratisierungsreaktion fördert. Unter dem Begriff "CO₂-Atmopshäre" wird dabei vorzugsweise eine Atmosphäre verstanden, welche mindestens 10 Vol-%, besonders bevorzugt mindestens 25 Vol-% und am meisten bevorzugt mindestens 50 Vol-% CO₂ beinhaltet.

Als Reaktoren für die Reaktivdestillation können alle dem Fachmann bekannten Destillations- oder Rektifikationsvorrichtungen eingesetzt werden. Der Katalysator kann dabei im Falle einer heterogenen Katalyse im inneren dieser Destillations- oder Rektifikationsvorrichtungen auf einem geeigneten Träger immobilisiert sein, beispielsweise durch die Verwendung von strukturierten Packungen, wie sie etwa unter der Bezeichnung "Katapak-S^{®}" von der Firma Sulzer-Chemtech erhältlich sind, oder durch die Verwendung von mit Katalysator beschichteten Thermoblechen, er kann aber auch, im Falle einer homogenen Katalyse, über einen geeigneten Einlass in das innere der Destillations- oder Rektifikationsvorrichtungen eingebracht werden. Weiterhin können grundsätzlich Packungskolonnen, welche Füllkörper wie etwa hohlzylindrische Füllkörper, beispielsweise RASCHIG-Ringe, INTOS-Ringe, PALL-Ringe, Maschen-drahtringe, Streckmantelringe, Wendelringe WILSON-Spiralringe oder PYM-Ringe, spulenförmige Füllkörper, wie etwa HALTMEIER-Rollen, sattelförmige Füllkörper, wie etwa BERL-Sättel, INTALOX-Sättel oder Maschendrahtsattel, kreuzförmige Füllköper, wie etwa Zwillingskörper, Propellerkörper oder Sternkörper, kastenförmige Füllkörper, wie etwa HELI-PAK-Körper oder OCTA-PAK-Körper, oder kugelförmige Füllkörper, wie etwa ENVI-PAK-Körper beinhalten, oder Bodenkolonnen eingesetzt werden, wobei die vorstehend beschriebenen Füllkörper zumindest teilweise mit Katalysator beschichtet sein können. Packungskolonnen haben im Vergleich zu Bodenkolonnen einen sehr geringen Flüssigkeitsinhalt. Dies ist zwar für die Rektifikation oft von Vorteil, da dadurch die Gefahr der thermischen Zersetzung der Substanzen vermindert wird. Für die Reaktivdestillation ist der geringe Flüssigkeitsinhalt von Packungskolonnen jedoch von Nachteil, insbesondere bei Reaktionen mit endlicher Reaktionsgeschwindigkeit. Um den gewünschten Umsatz bei langsamen Reaktionen zu erzielen, muss daher eine hohe Verweilzeit der Flüssigkeit im Apparat gewährleistet werden. Erfindungsgemäß besonders vorteilhaft kann daher der Einsatz von Bodenkolonnen sein. Diese haben einen hohen Flüssigkeitsinhalt, und zwar sowohl in der Zweiphasenschicht auf dem Boden als auch in den Ablaufschächten. Beide Anteile des Flüssigkeitsinhaltes können durch konstruktive Maßnahmen, d.h. durch die Gestaltung der Böden, gezielt verändert und an die jeweiligen Anforderungen der Reaktivdestillation angepasst werden. Weiterhin kann der Katalysator über die gesamte Länge, also vom Bodenbereich bis zum Kopfbereich, der Destillations- oder Rektifikationsvorrichtungen verteilt sein. Denkbar ist aber auch, dass der Katalysator lediglich in einem bestimmten Bereich, vorzugsweise in der unteren Hälfte, besonders bevorzugt im unteren Drittel und am meisten bevorzugt im unteren Viertel der Destillations- oder Rektifikationsvorrichtung lokalisiert ist.

Im Falle der vorstehend genannten Reaktivdestillation umfasst das erfindungsgemäße Verfahren vorzugsweise folgende Verfahrensschritte:
(a1) Herstellung von Hydroxypropionsäure, vorzugsweise von 2-Hydroxypropionsäure oder 3-Hydroxypropionsäure, am meisten bevorzugt von 3-Hydroxypropionsäure, aus einem biologischen Material unter Erhalt eines Hydroxypropionsäure, vorzugsweise 2-Hydroxypropionsäure oder 3-Hydroxypropionsäure, am meisten bevorzugt 3-Hydroxypropionsäure beinhaltenden wässrigen Phase P1, wobei die Herstellung der wässrigen Phase P1 folgende Verfahrensschritte umfasst:
   i) Herstellung, vorzugsweise fermentative Herstellung von Hydroxypropionsäure aus einem biologischen Material, besonders bevorzugt aus Kohlenhydraten, insbesondere aus Glukose, oder aus Glycerin, in einer wässrigen Zusammensetzung unter Erhalt einer Hydroxypropionsäure sowie Mikroorganismen beinhaltenden, wässrigen Phase,
   ii) gegebenenfalls Abtöten der Mikroorganismen, vorzugsweise durch Erhitzen dieser wässrigen Phase auf Temperaturen von mindestens 100°C, besonders bevorzugt mindestens 110°C und darüber hinaus bevorzugt mindestens 120°C für eine Dauer von mindestens 60 Sekunden, beispielsweise 10 Minuten und oder mindestens 30 Minuten, sowie
   iii) gegebenenfalls Abtrennen von Feststoffen, insbesondere von Mikroorganismen oder nicht umgesetztem biologischen Material, aus der wässrigen Phase, vorzugsweise mittels Sedimentation, Zentrifugation oder Filtration,
   iv) gegebenenfalls zumindest teilweises Abtrennen von Wasser aus der wässrigen Phase,
(a2) Dehydratisieren der Hydroxypropionsäure, vorzugsweise der 2-Hydroxypropionsäure oder 3-Hydroxypropionsäure, am meisten bevorzugt der 3-Hydroxypropionsäure, mittels einer Reaktivdestillation, vorzugsweise unter einer CO₂-Atmosphäre, unter Erhalt einer Acrylsäure beinhaltenden wässrigen Phase P2 als Sumpfprodukt, (a3) Aufreinigung des Acrylsäure beinhaltenden Sumpfproduktes durch eine Suspensionskristallisation oder eine Schichtkristallisation unter Erhalt einer gereinigten Phase.

Um eine möglichst hohe Verweilzeit der wässrigen Phase P1 in den Dehydratisierungsreaktoren zu gewährleisten, kann es vorteilhaft sein, neben der Destillations- oder Rektifikationsvorrichtung mindestens einen weiteren Reaktor einzusetzen, in dem zumindest ein Teil der in der wässrigen Phase P1 enthaltene Hydroxypropionsäure zur Acrylsäure umgesetzt wird, bevor die wässrige Phase P1 zur weiteren Umsetzung in die Destillations- oder Rektifikationsvorrichtung eingebracht wird.

Im Zusammenhang mit der vorstehend beschriebenen erfindungsgemäßen Verfahren, bei dem
eine Reaktivdestillation erfolgt, ist es vorgesehen, dass die Dehydratisierung mindestens in zwei Reaktoren durchgeführt wird, wobei der zweite ggf. jeder weitere Reaktor als Reaktivdestille ausgestaltet ist. Dabei wird in mindestens einen ersten Reaktor R1, der vorzugsweise nicht als Destillations- oder Rektifikationsvorrichtung ausge- bildet ist, die - vorzugsweise von Feststoffen befreite - wässrige Phase P1 in Gegenwart eines homogenen oder hete- rogenen Katalysators, vorzugsweise eine anorganische Säure, besonders bevorzugt Phosphorsäure, unter Erhalt eines
ersten, Acrylsäure beinhaltenden wässrigen Fluids F1_1, vorzugsweise einer ersten, Acrylsäure beinhaltenden wässrigen Phase P1_1 erhitzt, wobei dieses Erhitzen unter einem Druck Π1 erfolgt. Dieses Fluid F1_1 bzw. diese wässrige Phase P1_1 wird anschließend in einen ebenfalls einen homogenen oder heterogenen Katalysator beinhaltenden weiteren Reaktor R2, der vorzugsweise als Destillations- oder Rektifikationskolonne ausgebildet ist, eingebracht. In diesem Reaktor R2 erfolgt dann die weitere Dehydratisierung von noch vorhandener Hydroxypropionsäure durch Erhitzendes Fluids F1_1 in Gegenwart eines homogenen oder heterogenen Katalysators unter Erhalt eines Acrylsäure beinhaltenden Fluids F1_2, vorzugsweise einer Acrylsäure beinhaltenden wässrigen Phase P1_2 als Sumpfprodukt, wobei dieses Erhitzen unter einem Druck Π2 erfolgt, wobei der Druck Π2 allgemein verschieden zu dem Druck Π1 und in einer bevorzugten Ausgestaltung kleiner als Π1, vorzugsweise um mindestens 0,1 bar, besonders bevorzugt mindestens 1 bar, darüber hinaus bevorzugt mindestens 2 bar, ferner bevorzugt mindestens 4 bar, weiterhin bevorzugt mindestens
8 bar und darüber hinaus bevorzugt mindestens 10 bar kleiner als Π1 ist. In einer Ausgestaltung des erfindungsgemäßen Verfahrens liegt der druck des ersten Reaktors R1 in einem Bereich von 4,5 bis 25 bar, vorzugsweise in einem Bereich von 5 bis 20 bar und darüber hinaus bevorzugt in einem Bereich von 6 bis 10 bar und der Druck des weiteren Reaktors R2 in einem Bereich von 1 bis <4,5 bar und vorzugsweise in einem Bereich von 2 bis 4 bar. In einer Ausgestaltung des erfindungsgemäßen Verfahrens liegt der druck des ersten Reaktors R1 in einem Bereich von 4,5 bis 25 bar, vorzugsweise in einem Bereich von 5 bis 20 bar und darüber hinaus bevorzugt in einem Bereich von 6 bis 10 bar und der Druck des weiteren Reaktors R2 in einem Bereich von 0,01 bis <1 bar und vorzugsweise in einem Bereich von 0,1 bis 0,8 bar. Als Destillat wird in diesem Reaktionsgefäß R2 Wasser abgetrennt. Oftmals verlässt bei der Reaktivdestillation Wasser in einer Menge von 20 Gew.-% und mehr, vorzugsweise 40 Gew.-% und mehr und besonders bevorzugt 60 Gew.-% und mehr Überkopf. Im Sumpfprodukt der Reaktivdestillation verbleibt das restliche Wasser. Die vorstehenden Gew.-% basieren jeweils auf 100 Gew.-% der in die Reaktivkolonne eingefahren Komponente.

In diesem Zusammenhang ist es weiterhin bevorzugt, dass sowohl der Reaktor R1 als auch der Reaktor R2 vor dem Eintrag der wässrigen Phase P1, des Fluids F1_1 bzw. der wässrigen Phase P1_1 mit CO₂ begast werden, um, wie vorstehend beschrieben, Decarboxylierungsreaktionen während der Dehydratisierungsreaktion zu vermeiden.

Die Katalysatoren in den Reaktoren R1 und R2 können identisch oder verschieden sein, wobei die Höhe des Umsatzes und der Selektivität in den einzelnen Reaktionsgefäßen über die Verweilzeit im jeweiligen Katalysatorbett, über den Druck, über die Temperatur und, im Falle des zweiten Reaktionsgefäßes, über das Rücklaufverhältnis eingestellt werden können. Sofern ein homogener und meist flüssiger Katalysator, insbesondere eine anorganische Säure wie Phosphorsäure, eingesetzt wird, ist es bevorzugt, die mindestens die Hauptmenge dieses Katalysators in dem Reaktor R1 zuzugeben.

Die Dehydratisierung im Reaktor R1, bei dem es sich vorzugsweise um einen geschlossenen, druckfesten Reaktor handelt, liegt, je nach Art und Menge des verwendeten Katalysators und je nach dem im Reaktionsgefäß herrschenden Druck, vorzugsweise in einem Bereich von 80 bis 200°C, besonders bevorzugt in einem Bereich von 120 bis 160°C, während der Druck vorzugsweise in einem Bereich von 1 bis 10 bar, besonders bevorzugt von 3 bis 8 bar (abs.) liegt.

Das in diesem Reaktor R1 erhaltene Fluid F1_1 bzw. die wässrige Phase P1_1 wird dann in den als Destillations- oder Rektifikationskolonne ausgebildeten Reaktor R2 eingebracht. Dabei kann das Fluid F1_1 bzw. die wässrige Phase P1_1 sowohl in den Kopfbereich der Destillationskolonne, vorzugsweise in einen Bereich des oberen Drittels, besonders bevorzugt in einem Bereich des oberen Viertels und am meisten bevorzugt in einem Bereich des oberen Viertels der Destillations- oder Rektifikationskolonne, oder aber auch in einem Seitenbereich der Destillations- oder Rektifikationskolonne, vorzugsweise in einem Bereich zwischen dem unteren und dem oberen Drittel, besonders bevorzugt zwischen dem unteren und dem oberen Viertel der Destillations- oder Rektifikationskolonne, eingebracht werden.

Wird das Fluid F1_1 bzw. die wässrige Phase P1_1 in den Kopfbereich der Destillations- oder Rektifikationskolonne eingebracht, so wird in diesem Fall das Wasser vorzugsweise im Gegenstrom abdestilliert. Wird das Fluid F1_1 bzw. die wässrige Phase P1_1 im Seitenbereich der Destillations- oder Rektifikationskolonne eingebracht, so ist es bevorzugt, dass die Destillations- oder Rektifikationskolonne einen unteren Reaktionsbereich, in dem sich der Katalysator befindet, und einen an diesen Reaktionsbereich angrenzenden oberen Destillationsbereich, der frei ist von Katalysator, umfasst und dass das Fluid F1_1 bzw. die wässrige Phase P1_1 zwischen dem Reaktionsbereich und dem Destillationsbereich in die Destillationskolonne eingebracht wird.

Die Temperatur im Reaktor R2 liegt vorzugsweise im gleichen Bereich wie die Temperatur im Reaktor R1, während der Druck geringer ist, um eine Destillation des Wasser zu ermöglichen. Der Druck des Reaktors R2 ist vorzugsweise um 1 bis 5 bar gegenüber dm ersten Reaktor R1 abgesenkt.

Als Reaktionsgemisch bzw. als Phase P2, welches im Anschluss an die Dehydratisierung erhalten wird, wird vorzugsweise eine wässrige Acrylsäurelösung, die keine Katalysatorbestandteile enthält (eine solche wird im Falle einer heterogen katalysierten Dehydratisierung erhalten) oder aber eine wässrige Acrylsäurelösung, welche Katalysatoren beinhaltet (eine solche wird im Falle einer homogen katalysierten Dehydratisierung erhalten) als eine wässrige Phase P2 erhalten.

Wurde als Phase 1 eine wässrige, gegebenenfalls von Feststoffen befreite und gegebenenfalls zuvor in ihrem Wassergehalt reduzierte Fermentationslösung eingesetzt, so ist es bevorzugt, dass die im Verfahrensschritten (a2) erhaltene wässrige Phase P2 eine Zusammensetzung Z3 aufweist, beinhaltend
(Z3_1) 20 bis 95 Gew.-%, vorzugsweise 30 bis 90 Gew.-% und am meisten bevorzugt 50 bis 85 Gew.-% Acrylsäure, deren Salze oder Mischungen davon,
(Z3_2) 0 bis 5 Gew.-%, vorzugsweise 0,1 bis 2,5 Gew.-% und am meisten bevorzugt 0,5 bis 1 Gew.-% anorganische Salze,
(Z3_3) 0,1 bis 30 Gew.-%, vorzugsweise 0,5 bis 20 Gew.-% und am meisten bevorzugt 1 bis 10 Gew.-% von Acrylsäure verschiedene, organische Verbindungen, insbesondere Hydroxypropionsäuren,
(Z3_4) 0 bis 50 Gew.-%, vorzugsweise 1 bis 40 Gew.-%, vorweise 5 bis 20 Gew.-% und am meisten bevorzugt 1 bis 10 Gew.-% Zellen, sowie
(Z3_5) 1 bis 90 Gew.-%, vorzugsweise 50 bis 80 Gew.-% und am meisten bevorzugt 10 bis 70 Gew.-% Wasser,
wobei die Summe der Komponenten (Z3_1) bis (Z3_5) 100 Gew.-% beträgt. Insbesondere dann, wenn die Dehydratisierung der Hydroxypropionsäure mittels des vorstehend beschriebenen Verfahrens der Reaktivdestillation durchgeführt wurde, ist es bevorzugt, dass die Acrylsäure beinhaltende Zusammensetzung Z3, welche als Sumpfprodukt bei der Reaktivdestillation erhalten wird, weniger als 25 Gew.-%, besonders bevorzugt weniger als 10 Gew.-% und am meisten bevorzugt weniger als 5 Gew.-% Wasser, jeweils bezogen auf das Gesamtgewicht der Zusammensetzung Z3, beinhaltet.

Weiterhin kann es vorteilhaft sein, dass das Phase P1 oder das Phase P2 vor Durchführung des Verfahrensschrittes (a2) bzw. (a3) durch ein Adsorptionsverfahren, insbesondere durch Reinigung mit einem handelsüblichen Filter, insbesondere, einem Aktivkohlefilter, aufgereinigt wird.

Sofern bisher noch keine Feststoffe wie beispielsweise Zellen abgetrennt worden sind, kann es hilfreich sein, vor Durchführung der Kristallisation im Verfahrensschritt (a3) diese Feststoffe durch die vorstehend beschriebenen Filtrationsverfahren, beispielsweise durch Ultrafiltration, abzutrennen.

Im Verfahrensschritt (a3) des erfindungsgemäßen Verfahrens wird nun die in Phase P2 enthaltene Acrylsäure durch Kristallisation, vorzugsweise durch eine Suspensionskristallisation oder eine Schichtkristallisation, unter Erhalt einer gereinigten Phase aufgereinigt, wobei eine kontinuierlich durchgeführte Suspensionskristallisation besonders bevorzugt ist. Die Kristallisation kann in einer Ausgestaltung des erfindungsgemäßen Verfahrens bereits mit der wasserreichen Acrylsäure und Hydroxypropionsäure beinhaltenden wässriger Phase P2 (bzw. Phase F1_1) erfolgen, wie sie nach der Dehydratisierung in dem ersten Reaktor R1 anfällt. In einer anderen Ausgestaltung des erfindungsgemäßen Verfahrens erfolgt die Kristallisation mit der aus dem weiteren Reaktor stammenden im Vergleich zur Phase P2 wasserärmeren Phase F1_2. Oftmals beinhaltet die Phase P2 neben Acrylsäure und Hydroxypropionsäure mehr als 30 Gew.-%, vorzugsweise mehr als 50 Gew.-% und besonders bevorzugt mehr als 70 Gew.-% Wasser. Hingegen weist die Phase F1_2 neben Acrylsäure und Hydroxypropionsäure oft weniger als 30 Gew.-%, vorzugsweise weniger als 20 Gew.-% und besonders bevorzugt weniger als 10 Gew.-%.

Die Abtrennung der Kristalle von der Mutterlauge kann im Falle der Suspensionskristallisation durch eine Waschkolonne erfolgen. Für den erfolgreichen Betrieb einer Waschkolonne ist es vorteilhaft, dass die zu waschenden Kristalle eine hinreichende Härte aufweisen und eine bestimmte schmale Größenverteilung aufweisen, um eine entsprechende Porösität und Stabilität des entstehenden gepackten oder ungepackten Filterbettes zu gewährleisten.

Die Suspensionskristallisation kann vorteilhafterweise in einem Rührkesselkristallisator, Kratzkristallisator, Kühlscheibenkristallisator, Kristallisierschnecke, Trommelkristallisator, Rohrbündelkristallisator oder dergleichen realisiert werden. Insbesondere können die in WO-A-99/14181 genannten Kristallisationsvarianten für den genannten Zweck herangezogen werden. Von besonderem Vorteil sind hier wiederum insbesondere solche Kristallisatoren, die kontinuierlich betrieben werden können. Vorzugsweise sind dies die Kühlschreibenkristallisatoren oder der Kratzkühler (Disseration Poschmann, S. 14). Ganz besonders bevorzugt wird zur Kristallisation ein Kratzkühler eingesetzt.

Im Prinzip kann für das erfindungsgemäße Verfahren jedwede Waschkolonne eingesetzt werden, welche die kontinuierliche Fahrweise der erfindungsgemäßen Aufreinigung zulässt. Bei einer üblichen Ausführungsform wird die Suspension in einer hydraulischen Waschkolonne im oberen Teil der Kolonne aufgegeben. Die Mutterlauge wird über einen Filter aus der Waschkolonne abgezogen, wobei sich ein dicht gepacktes Kristallbett bildet. Das Kristallbett wird von der Mutterlauge in Richtung des Bodens der Kolonne durchströmt und durch den Strömungswiderstand nach unten gedrückt. Am Boden der Kolonne befindet sich eine bewegte vorzugsweise rotierende Kratzvorrichtung oder Kratzer, die aus dem dicht gepackten Kristallbett und der am unteren Teil der Waschkolonne eingebrachten Waschschmelze wieder eine Suspension erzeugt. Diese Suspension wird vorzugsweise durch einen Aufschmelzer, vorzugsweise einen Wärmetauscher, abgepumpt und aufgeschmolzen. Ein Teil der Schmelze kann z. B. als Waschschmelze dienen; diese wird dann in die Kolonne zurückgepumpt und wäscht vorzugsweise das in entgegengesetzter Richtung wandernde Kristallbett aus, d. h. die kristallisierte Acrylsäure wird im Gegenstrom von der zurückgeführten Acrylsäure gewaschen. Vor dem Hintergrund einer Ausbeutesteigerung ist eine Rückführung abgetrennter Mutterlauge besonders vorteilhaft. Die Waschschmelze bewirkt einerseits ein Waschen der Kristalle, andererseits kristallisiert die Schmelze auf den Kristallen zumindest teilweise aus. Die freiwerdende Kristallisationsenthalpie erwärmt das Kristallbett im Waschbereich der Kolonne. Dadurch wird ein dem Schwitzen der Kristalle analoger Reinigungseffekt erzielt.

Damit wird eine Aufreinigung zum einen durch das Waschen der Oberfläche der Acrylsäure mit aufgeschmolzener - und damit bereits gereinigter Acrylsäure bewirkt, zum anderen wird durch die Kristallisation der aufgeschmolzenen, gereinigten Acrylsäure auf den bereits vorhandenen Acrylsäurekristallen ein Ausheilen bzw. Ausschwitzen von Verunreinigungen erreicht. Dieses erlaubt eine besonders hochreine Herstellung von Acrylsäure.

Grundsätzlich ist es jedoch kann die Wäsche der durch Suspensionskristallisation erhaltenen Kristalle jedoch auch auf andere Weise als die Gegenstromwäsche in einer Waschkolonne erfolgen. So können die Kristalle beispielsweise auf einem Bandfilter gewaschen werden, nach dem sie mittels einer geeigneten Trennvorrichtung, etwa einem Filter, von der Mutterlauge abgetrennt wurden.

Gemäß einer weiteren Ausführungsform des Verfahrens wird die wässrige Phase P2, vorzugsweise die Phase F1_2, bis maximal an die Temperatur T des Trippel-Eutektischen Punktes einer Zusammensetzung aus Acrylsäure, Wasser und Hydroxypropionsäure abgekühlt, vorzugsweise bis auf einer Temperatur, die höchstens 6 Kelvin, besonders bevorzugt höchstens 3 Kelvin und am meisten bevorzugt höchstens 1 Kelvin über der Temperatur des Trippel-Eutektischen Punktes liegt.

Vorzugsweise wird bei der Aufreinigung mittels Kristallisation eine erste Kristallphase erhalten, beinhaltend
- mindestens 30 Gew.-% Acrylsäure, vorzugsweise mindestens 40 Gew.-% und am meisten bevorzugt mindestens 50 Gew.-% Acrylsäure,
- mindestens 30 Gew.-%, vorzugsweise mindestens 40 Gew.-% und am meisten bevorzugt mindestens 49 Gew.-% Wasser, sowie
- höchstens 10 Gew.-%, vorzugsweise höchstens 5 Gew.-% und am meisten bevorzugt höchstens 1 Gew.-% Hydroxypropionsäure.

Die Aufreinigung der Acrylsäure aus der wässrigen Phase P2 mittels Kristallisation kann ein-, zwei-, drei- oder mehrstufig erfolgen. Bei einer zweistufigen Kristallisation werden die in der ersten Kristallisationsstufe erhaltenen Kristalle von der zurückbleibenden Mutterlauge abgetrennt, aufgeschmolzen und in einer zweiten Kristallisationsstufe erneut kristallisiert.

Insbesondere dann, wenn wässrige Phase P2, eine Zusammensetzung Z3 eingesetzt wird, welche, neben Acrylsäure, von Acrylsäure verschiedenen organischen Verbindungen, wie insbesondere im Verfahrensschritt (a2) nicht dehydratisierte Hydroxypropionsäure, sowie gegebenenfalls anorganische Salze, mehr als 5 Gew.-%, besonders bevorzugt mehr als 10 Gew.-% und am meisten bevorzugt mehr als 25 Gew.-% Wasser beinhaltet, ist es gemäß einer besonderen Ausführungsform des erfindungsgemäßen Verfahrens bevorzugt, die Kristallisation im Verfahrensschritt (a3) als eine mindestens zweistufige und vorzugsweise mindestens dreistufige Kristallisation auszugestalten. Bei der mindestens zweistufigen Kristallisation ist es bevorzugt, dass in mindestens zwei, vorzugsweise aufeinander folgenden Stufen sich dem jeweiligen Eutektikum der Hauptkomponenten einer solchen Stufe, die in der Regel mit 1 und mehr Gew.-% in dem jeweiligen Feed enthalten sind, durch Variation der Kristallisationsbedingungen, vorzugsweise Senken der Temperatur, angenähert wird. Wenn in drei und mehr Stufen kristallisiert wird, ist es bevorzugt, dass vor der Stufe, in der das Eutektikum der jeweiligen Hauptkomponenten des Feeds, vorzugsweise Wasser und Acrylsäure, durch Variation der Kristallisationsbedingungen, vorzugsweise Senken der Temperatur, unterschritten wird, mindestens eine, vorzugsweise mindestens zwei Stufen erfolgen, in denen sich dem jeweiligen Eutektikum der Hauptkomponenten durch Variation der Kristallisationsbedingungen, vorzugsweise Senken der Temperatur, angenähert wird. Bei den vorstehen Stufen ist es bevorzugt, eine Abkühlrate in einem Bereich von 0,01 bis 10 K/min, vorzugsweise in einem Bereich von 0,05 K/min bis 5 K/min und besonders bevorzugt in einem Bereich von 0,1 bis 1 K/min zu verwenden.

So ist es weiterhin bevorzugt, wenn der Verfahrensschritt (a3) die folgenden Teilschritte beinhaltet, die vorzugsweise unmittelbar aufeinander folgen:
(a3_1) Kristallisation, vorzugsweise Suspensions- oder Schichtkristallisation, am meisten bevorzugt Suspensionskristallisation, der wässrigen Phase P2, in einer ersten Kristallisationsstufe unter Erhalt einer Kristallphase K1 und einer Mutterlauge M1, wobei die Kristallphase K1:
   - 5 bis 60 Gew.-%, besonders bevorzugt 10 bis 55 Gew.-% und am meisten bevorzugt 15 bis 50 Gew.-% Acrylsäure,
   - 39,9 bis 95 Gew.-%, besonders bevorzugt 44,6 bis 90 Gew.-% und am meisten bevorzugt 80 bis 99,5 Gew.-% Wasser, sowie
   - 0,1 bis 10 Gew.-%, besonders bevorzugt 0,4 bis 8 Gew.-% und am meisten bevorzugt 1 bis 6 Gew.-% von Wasser und Acrylsäure verschiedene Nebenprodukte umfasst,
   und wobei die Summe der Gewichtsmengen von Acrylsäure, Wasser und Nebenprodukten 100 Gew.-% beträgt,
(a3_2) Abtrennen der Kristallphase K1 von der Mutterlauge M1, vorzugsweise mittels einer Waschkolonne, wobei die Kristalle vorzugsweise in der vorstehend beschriebenen Art und Weise einer Kristallwäsche unterzogen werden,
(a3_3) Aufschmelzen der Kristallphase K1 aus der ersten Kristallisationsstufe,
(a3-4) Erneute Kristallisation, vorzugsweise Suspensions- oder Schichtkristallisation, am meisten bevorzugt Suspensionskristallisation, der aufgeschmolzenen Kristallphase in einer zweiten Kristallisationsstufe unter Erhalt einer Kristallphase K2 und einer Mutterlauge M2, wobei die Kristallphase K2:
   - 8 bis 35 Gew.-%, besonders bevorzugt 1 bis 28 Gew.-% und am meisten bevorzugt 0,4 bis 19,75 Gew.-% Acrylsäure,
   - mindestens 60 Gew.-%, besonders bevorzugt mindestens 70 Gew.-% und am meisten bevorzugt 80 bis 99,5 Gew.-% Wasser, sowie
   - maximal 5 Gew.-%, besonders bevorzugt maximal 2 Gew.-% und am meisten bevorzugt 0,1 bis 0,25 Gew.-% von Wasser und Acrylsäure verschiedene Nebenprodukte,
   wobei die Summe der Gewichtsmengen von Acrylsäure, Wasser und Nebenprodukten 100 Gew.-% beträgt,
(a3_5) Abtrennen der Kristallphase K2 von der Mutterlauge M2, vorzugsweise mittels einer Waschkolonne,
(a3-6) Kristallisation, vorzugsweise Suspensions- oder Schichtkristallisation, am meisten bevorzugt Suspensionskristallisation, der Mutterlauge M2 in einer dritten Kristallisationsstufe unter Erhalt einer Kristallphase K3 und einer Mutterlauge M3, wobei die Kristallphase K3:
   - mindestens 40 Gew.-%, besonders bevorzugt mindestens 50 Gew.-% und am meisten bevorzugt 55 bis 70 Gew.-% Acrylsäure,
   - maximal 70 Gew.-%, besonders bevorzugt maximal 57,5 Gew.-% und am meisten bevorzugt 45 bis 57,5 Gew.-% Wasser, sowie
   - maximal 5 Gew.-%, besonders bevorzugt maximal 2,5 Gew.-% und am meisten bevorzugt 0 bis 1,5 Gew.-% von Wasser und Acrylsäure verschiedene Nebenprodukte,
   wobei die Summe der Gewichtsmengen von Acrylsäure, Wasser und Nebenprodukten 100 Gew.-% beträgt, sowie
(a3_7) Abtrennen der Kristallphase K3 von der Mutterlauge M3, vorzugsweise mittels einer Waschkolonne, wobei die Kristalle vorzugsweise in der vorstehend beschriebenen Art und Weise einer Kristallwäsche unterzogen werden, wobei letztendlich einer gereinigte Phase beinhaltend Acrylsäurekristalle erhalten wird.

Bei der vorstehend beschriebenen besonderen Ausführungsform des erfindungsgemäßen Verfahrens mit dreistufiger Kristallisationsstufe im Verfahrensschritt (a3) kann es besonders vorteilhaft sein, die in der ersten Kristallisationsstufe erhaltene Mutterlaufe, welche noch größere Mengen an nicht dehydratisierter Hydroxypropionsäure beinhaltet, in den Verfahrensschritt (a2) zum Zwecke einer möglichst vollständigen Dehydratisierung zurückzuführen.

Ausführungsformen zur Suspensionskristallisation mit nachgeschalteter Wäsche der Kristalle in einer hydraulischen oder mechanischen Waschkolonne sind dem Buch "Melt crystallization technology" von G. F. Arkenbout, Technomic Publishing Co. Inc., Lancaster-Basel (1995), S. 265 bis 288 sowie dem sich auf die Niro-Gefrierkonzentration zur Abwasservorkonzentrierung richtenden Artikel in Chemie-Ingenieurtechnik (72) (1Q/2000), 1231 bis 1233 zu entnehmen.

Als Waschflüssigkeit kann je nach Einsatzzweck eine dem Fachmann geläufige Waschflüssigkeit benutzt werden (bei wässrigen Lösungen z. B. Wasser). Ganz besonders bevorzugt dient zum Waschen der kristallisierten Acrylsäure, des kristallisierten Wassers bzw. der kristallisierten Mischung aus Wasser und Acrylsäure, wie schon angedeutet, eine Teilmenge der aufgeschmolzenen Kristalle derselben. Durch diese Maßnahme wird zum einen gewährleistet, dass zur Produktion hochreiner Produkte kein weiterer Stoff in das System eingeführt werden muss, zum anderen dienen die aufgeschmolzenen Kristalle aber auch zum Zurückdrängen der Mutterlaugenfront in der Waschkolonne und üben gleichzeitig auf die Kristalle einen reinigenden Effekt analog zum Schwitzen aus. Ein Produktverlust findet dabei nicht statt, da die Waschflüssigkeit auf den zu waschenden Kristallen wiederum auskristallisiert und sich so im Produkt wieder findet (z. B. Prospekt der Niro Process Technology B.V. Crystallization and wash column separations set new standards in purity of chemical compounds).

In einer alternativen Ausgestaltung erfolgt die Bildung der Kristalle in mindestens einem der Verfahrensschritte (a3_1), (a3_4) und a3_6) in einer Schicht. Eine Schichtkristallisation erfolgt gemäß dem Verfahren der Sulzer AG, Schweiz (http://www.sulzerchemtech.com). Ein geeigneter Schichtkristallisator und die Vorgehensweise bei der Schichtkristallisation ist beispielsweise in WO-A-00/45928 beschrieben, die hiermit als Referenz eingeführt wird und deren Offenbarungsgehalt hinsichtlich der Schichtkristallisation einen Teil der Offenbarung der vorliegenden Erfindung bildet.

Letztendlich kann durch das erfindungsgemäße Verfahren zur Herstellung von Acrylsäure eine Acrylsäure mit einer Reinheit von mindestens 50 Gew.-%, insbesondere mindestens 70 Gew.-%, vorzugsweise mindestens 90 Gew.-%, hergestellt werden.

Ein Verfahren zur Herstellung von Polyacrylaten sieht vor, dass eine Acrylsäure, erhältlich aus dem erfindungsgemäßen Verfahren polymerisiert, vorzugsweise radikalisch polymerisiert, wird.

Die radikalische Polymerisation der Acrylsäure erfolgt durch das den Fachmann bekannte Polymerisationsverfahren. Wenn es sich bei den Polymeren um vernetzte, teilneutralisierte Polyacrylate handelt, so wird hinsichtlich der genauen Vorgehensweise auf das 3. Kapitel (Seite 69ff) in "Modern Superabsorbent Polymer Technology", F. L. Buchholz und A. T. Graham (Herausgeber) in, Wiley-VCH, New York, 1998 verwiesen.

Die erfindungsgemäße Vorrichtung zur Herstellung von Acrylsäure beinhaltet folgende fluidleitend miteinander verbundene Einheiten:
- eine Syntheseeinheit zur Herstellung von Hydroxypropionsäure, gefolgt von,
- einer Dehydratisierungsstufe zur Umwandlung der Hydroxypropionsäure in Acrylsäure, wobei die Dehydratisierungsstufe mindestens zwei fluidleitend miteinander verbundene Reaktoren R1 und R2 aufweist, von denen einer (R1) mit einem Druck Π1 und der andere (R2) mit einem Druck Π2 belastbar ist, wobei der Druck Π2 von Druck Π1 verschieden ist, gefolgt von
- Aufreinigungseinheit der Acrylsäure beinhaltenden Phase P2 durch eine Suspensionskristallisation oder eine Schichtkristallisation unter Erhalt einer gereinigten Phase, insbesondere
   eine Synthetisierungseinheit zur Herstellung von Hydroxypropionsäure aus einem biologischen Material unter Erhalt eines Hydroxypropionsäure beinhaltenden Fluids F1, insbesondere einer wässrigen Phase P1,
   eine Dehydratisierungsstufe der Hydroxypropionsäure unter Erhalt eines Acrylsäure beinhaltenden Fluids F2, insbesondere einer wässrigen Phase P2, sowie
   eine Aufreinigungseinheit des Acrylsäure beinhaltenden Fluids F2 durch eine Suspensionskristallisation oder eine Schichtkristallisation unter Erhalt einer gereinigten Phase.

Unter "fluidleitend" wird erfindungsgemäß verstanden, dass Gase oder Flüssigkeiten, Suspension eingeschlossen, oder deren Mischungen durch entsprechende Leitungen geführt werden. Hierzu lassen sich insbesondere Rohrleitungen, Pumpen oder dergleichen einsetzen.

Mit Hilfe der Synthetisierungseinheit wird Hydroxypropionsäure aus einem regenerativen Rohstoff synthetisiert, welche anschließend mittels der Dehydratisierungsstufe zu Acrylsäure umgesetzt wird. Die Acrylsäure wird dann im Rahmen einer Schichtkristallisation bzw. einer Suspensionskristallisation aufgereinigt. Die erfindungsgemäße Vorrichtung kann neben einem herkömmlichen Bioreaktor auch eine Sterilisationseinheit, in der nach Beendigung der Fermentation die in der Fermenatationsbrühe enthaltenen Mikroorganismen abgetötet werden können, eine Filtrationseinheit, in der in der Fermentationsbrühe enthaltene Feststoffe durch Filtration, vorzugsweise durch Ultrafiltration abgetrennt werden können, eine Protonierungseinheit, in der zumindest ein Teil der gegebenenfalls als Salz vorliegenden Hydroxypropionsäure in ihre Säureform überführt werden kann, und/oder eine Entwässerungseinheit, in der zumindest ein Teil des Wasser abgetrennt werden kann, umfassen. Dabei können diese Einheiten sowohl zwischen der Synthetisierungseinheit und der Dehydratisierungsstufe als auch zwischen der Dehydratisierungsstufe und der Aufreinigungseinheit lokalisiert sein, wobei zumindest die Sterilisationseinheit und die Filtrationseinheit vorzugsweise zwischen der Synthetisierungseinheit und der Dehydratisierungsstufe lokalisiert sind.

Gemäß einer bevorzugten Ausführungsform der erfindungsgemäßen Vorrichtung ist die Dehydratisierungsstufe als Druckreaktor ausgebildet, wobei es weiterhin besonders bevorzugt ist, dass die Dehydratisierungsstufe eine Destillations- oder Rektifikationsvorrichtung umfasst, welche einen der eingangs beschriebenen Dehydratisierungskatalysatoren beinhaltet. Mittels einer solchen Dehydratisierungsstufe kann die im Zusammenhang mit dem erfindungsgemäßen Verfahren zur Herstellung von Acrylsäure beschriebene Reaktivdestillation durchgeführt werden. Als Vorrichtung für diesen Zweck eignen sich alle bekannten Bauformen von Destillationskolonnen, insbesondere Packungskolonnen und Bodenkolonnen.

Die Dehydratisierungsstufe weist mindestens zwei fluidleitend miteinander verbundene Dehydratisierungsreaktoren R1 und R2 auf, welche alle einen der eingangs beschriebenen Dehydratisierungskatalysatoren beinhalten, von denen einer mit einem Druck P1 und der andere mit einem Druck Π2 belastbar ist, wobei die beiden Drücke verschieden von einander sind und bevorzugt der Druck Π2 kleiner ist als Π1. Die bevorzugten Druckdifferenzen sind diejenigen Druckdifferenzen, die bereits Eingangs im Zusammenhang mit dem erfindungsgemäßen Verfahren beschrieben worden sind. Der Reaktor R2 ist als Destillations- oder Rektifikationsvorrichtung ausgebildet und der Reaktor R1 nicht. Diese Destillations- oder Rektifikationsvorrichtung ist derart mit der Aufreinigungseinheit verbunden, dass das bei der Destillations- oder Rektifikation erhaltene Sumpfprodukt in die Aufreinigungseinheit überführt werden kann.

Mittels des Dehydratisierungsreaktors R1, in welchen die vorzugsweise von Feststoffen befreite und gegebenenfalls mindestens teilweise entwässerte Fermentationslösung eingebracht wird, wird zumindest ein Teil der Hydroxypropionsäure zur Acrylsäure umgesetzt. Der erste Dehydratisierungsreaktor R1 ist dabei vorzugsweise derart mit dem zweiten Dehydratisierungsreaktor R2 verbunden, dass die im ersten Reaktor R1 erhaltene wässrige Lösung beinhaltend Hydroxypropionsäure, Acrylsäure und Wasser in den Kopfbereich des zweiten Reaktors, vorzugsweise in einen Bereich des oberen Drittels, besonders bevorzugt in einem Bereich des oberen Viertels und am meisten bevorzugt in einem Bereich des oberen Viertels des Reaktors R2, oder aber auch in einem Seitenbereich des Reaktors R2, vorzugsweise in einem Bereich zwischen dem unteren und dem oberen Drittel, besonders bevorzugt zwischen dem unteren und dem oberen Viertel des Reaktors R2, eingebracht werden. Mittels des Reaktors R1 kann dem Umstand Rechnung getragen werden, dass für eine Reaktivdestillation der geringe Flüssigkeitsinhalt von den für die Rektifikation eingesetzten Packungskolonnen von Nachteil ist, insbesondere bei Reaktionen mit endlicher Reaktionsgeschwindigkeit. Um den gewünschten Umsatz bei langsamen Reaktionen zu erzielen, muss eine hohe Verweilzeit der Flüssigkeit im Apparat gewährleistet werden. Man behilft sich dadurch, dass neben der Kolonne ein oder mehrere externe Behälter (Reaktor R1, gegebenenfalls können auch weitere externe Behälter (Reaktoren, R1', R1" usw. vorhanden sein) angebracht werden, in denen ein Großteil der eigentlichen Reaktion stattfindet. Die Rückführung der Flüssigkeit aus diesen Behältern in die Kolonne erfordert jeweils eine Unterteilung der Packung und eine aufwendige Flüssigkeitsverteilung, so dass für die Reaktivdestillation vorteilhafterweise Bodenkolonnen mit vergleichsweise großen Flüssigkeitsinhalten eingesetzt werden. Bodenkolonnen haben einen hohen Flüssigkeitsinhalt, und zwar sowohl in der Zweiphasenschicht auf dem Boden als auch in den Ablaufschächten. Beide Anteile des Flüssigkeitsinhaltes können durch konstruktive Maßnahmen, d.h. durch die Gestaltung der Böden bzw. die Höhe der Wehre, gezielt verändert und an die Reaktivdestillation angepasst werden. Beim Entwurf von geeigneten Einbauten für eine reaktive Bodenkolonne spielen folgende Gesichtspunkte eine wichtige Rolle: (i) Realisierung sehr hoher Flüssigkeitsinhalte, d.h. Verweilzeiten; (ii) beliebige Verteilung des Hold-up über die Kolonnenhöhe; (iii) Eignung sowohl für homogene als auch für heterogene Katalyse; (iv) leichte Austauschbarkeit des Katalysators; (v) einfaches Scale-up vom Labor auf großtechnische Kolonnen.

Mittels der Aufreinigungseinheit kann die die Acrylsäure beinhaltende Phase P2 durch eine Suspensionskristallisation oder eine Schichtkristallisation unter Erhalt einer gereinigten Phase aufgereinigt werden.

Gemäß einer besonders bevorzugten Ausführungsform der erfindungsgemäßen Vorrichtung umfasst die Aufreinigungseinheit folgende, fluidleitend miteinander verbundene Bestandteile:
(δ1) einen ersten Kristallisationsbereich, einen zweiten Kristallisationsbereich einen dritten Kristallisationsbereich, einen ersten Trennbereich, einen zweiten Trennbereich, einen dritten Trennbereich, mindestens einen Aufschmelzer, und mindestens sechs Führungen,
(δ2) der erste Kristallisationsbereich ist mit dem ersten Trennbereich über eine erste Führung verbunden,
(δ3) der erste Trennbereich ist mit dem mindestens einen Aufschmelzer über eine zweite Führung verbunden,
(δ4) der mindestens eine Aufschmelzer ist mit dem zweiten Kristallisationsbereich über eine dritte Führung verbunden,
(δ5) der zweite Kristallisationsbereich ist mit dem zweiten Trennbereich über eine vierte Führung verbunden,
(δ6) der zweite Trennbereich ist mit dem dritten Kristallisationsbereich über eine fünfte Führung verbunden,
(δ7) der dritte Kristallisationsbereich ist mit dem dritten Trennbereich über eine sechste Führung verbunden.

Dabei ist es bevorzugt, dass die Dehydratisierungsstufe vorzugsweise unmittelbar mit dem ersten Kristallisationsbereich fluidleitend verbunden ist, wobei im Falle einer Reaktivdestillations- oder Reaktivrektifikationskolonne in der Dehydratisierungsstufe diese Kolonnen vorzugsweise dergestalt mit der ersten Kristallisationseinheit verbunden sind, dass das anfallende Sumpfprodukt in die Kristallisationseinheit überführt werden kann. Diese mehrer aufeinander folgende Kristallisationsbereiche aufweisende erfindungsgemäße Kristallisationsvorrichtung kann sich in einer erfindungsgemäßen Ausgestaltung an den ersten Reaktor R1 anschließen. So können sehr reine wässrige Acrylsäurelösungen erhalten werden, so dass sich an die Kristallisationsvorrichtung direkt eine Polymerisationsvorrichtung anschließen kann, in der diese Acrylsäurelösung polymerisiert wird.

Werden als Kristallisationsbereiche Suspensionskristallisatoren und als Trennbereiche Waschkolonnen eingesetzt, so kann mit dieser besonderen Ausfürhungsform der erfindungsgemäßen Vorrichtung zunächst die in der Dehydratisierungsstufe erhalte, Acrylsäure beinhaltende Phase P2 im ersten Kristallisationsbereich unter Erhalt von Wasser- und Acrylsäurekristallen kristallisiert werden. Diese können dann in einer Waschkolonne (erste Trenneinheit) gewaschen und von der zurückbleiben den Mutterlaufe, welche vor allem noch Hydroxypropionsäure beinhaltet, abgetrennt werden. Die abgetrennten Kristalle, welche vor allem Wasser und Acrylsure beinhalten, können dann in dem mindestens einen Aufschmelzer geschmolzen und dann im zweiten Kristallisationsbereich erneut kristallisiert werden. Die in diesem zweiten Kristallisationsbereich anfallenden, vor allem auf Wasser basierenden Kristalle können in einer weiteren Waschkolonne (zweite Trenneinheit) von der Mutterlauge abgetrennt werden. Die zurückbleibende Mutterlauge kann dann in einem dritten Kristallisationsbereich unter Erhalt von im wesentlichen auf Acrylsäure basierenden Kristallen kristallisiert werden, wobei diese Kristalle dann in einer weiteren Waschkolonne (dritte Trenneinheit) gewaschen und von der zurückbleibenden Mutterlauge abgetrennt werden können.

Gemäß einer weiterhin vorteilhaften Ausgestaltung der vorstehend beschriebenen besonders bevorzugten Ausführungsform der erfindungsgemäßen Vorrichtung, bei der drei Kristallisationsbereiche vorhanden sind, sind die erste Trenneinheit und die dritte Trenneinheit mit einem zweiten bzw. einem dritten Aufschmelzer fluidleitend verbunden. Mit diesem zweiten bzw. dritten Aufschmelzer können die in der ersten bzw. dritten Trenneinheit von der Mutterlauge abgetrennten Kristalle zumindest teilweise aufgeschmolzen und die so erhaltene Kristallschmelze zur Gegenstromwäsche der in den jeweiligen Trenneinheiten vorliegenden Kristallphasen eingesetzt werden, wie dies beispielsweise in der DE-A-101 49 353 beschrieben ist.

Aufgrund der Reinigungsweise wird die so hergestellte Acrylsäure besonders schonend behandelt, wodurch ihre Qualität verbessert wird. Die Aufreinigungsvorrichtung ist in der Lage, aus einem vergleichsweise verunreinigten Acrylsäurestrom, welcher vor allem Wasser und Hydroxypropionsäure als Nebenprodukte beinhaltet, von rund 12 Gewichtsprozent sehr reine Acrylsäure mit Reinheitsgraden von über 60 Gewichtsprozent, insbesondere von über 99,5 Gewichtsprozent, zu gewinnen. Es ist nach der Erfindung möglich, eine Acrylsäurestrom mit 50 Gewichtsprozent bis 95 Gewichtsprozent Acrylsäure, vorzugsweise 75 bis 90 Gewichtsprozent Acrylsäure effizient aufzureinigen.

Die effektive Aufreinigung erlaubt, weitere thermische Behandlungsmethoden, insbesondere eine Destillation oder Verdampfung, zu reduzieren, so dass die thermische Belastung der Acrylsäure bzw. daraus gebildeter Acrylsäure verringert wird. Hierdurch wird die Qualität der Acrylsäure bzw. der Acrylsäure verbessert.

Zur weiteren Steigerung der Reinheit der Acrylsäure weist die Vorrichtungseinheit eine separate Reinigungsvorrichtung auf. Diese separate Reinigungsvorrichtung kann zur weiteren Aufreinigung des Endprodukts, insbesondere zur Weiteraufreinigung der den Aufschmelzer verlassenden Acrylsäure, eingesetzt werden.

Zur Steigerung der Ausbeute ist es besonders bevorzugt, den ersten Trennbereich mit dem Dehydratisierungsreaktor zu verbinden, so dass die im Trennbereich anfallenden Mutterlauge zwecks möglichst vollständiger Umsetzung der Hydroxypropionsäure wieder in die Dehydratisierungsstufe eingebracht werden kann.

Bei dem erfindungsgemäßen Verfahren zur Aufreinigung von Acrylsäure herrschen in den Trennbereichen Temperaturen im Bereich von -20 bis +20 °C, vorzugsweise von -10 bis +13 °C bei einem Druck von 1 bis 10 bar. Es ist bevorzugt, dass in dem unteren Bereich der Trennbereiche eine geringere Temperatur und ein geringerer Druck herrschen als im oberen Bereich der Trennbereiche. Vorzugsweise herrschen in dem unteren Bereich der Trennbereiche -20 bis < 12 °C bei einem Druck von 1 bis 2 bar. Im oberen Bereich der Trennbereiche herrschen eine Temperatur von mindestens 12 °C und ein Druck von 1 bis 10 bar, vorzugsweise 3 bis 7 bar. Vorteilhafterweise herrschen in den Kristallisationsbereichen Temperaturen im Bereich von -20 bis 20 °C, vorzugsweise von -12 bis 13 °C bei einem Druck von 0,5 bis 10 bar, vorzugsweise von 0,8 bis 2 bar. In dem mindestens einen Aufschmelzer kann eine Temperatur im Bereich von 10 bis 50 °C, vorzugsweise von 11 bis 10 °C bei einem Druck von 1 bis 10 bar, vorzugsweise 3 bis 7 bar, herrschen.

In den Führungen herrschen insbesondere Temperatur- und Druckverhältnisse, die einen sicheren und störungsfreien Transport der Acrylsäure und den diese gegebenenfalls begleitenden Stoffe in diesen Führungen erlauben.

Die Vorrichtung erlaubt es, von einer verhältnismäßig verunreinigten Acrylsäure auszugehen, welche gegebenenfalls noch große Wasser- und Hydroxypropionsäure-Mengen beinhaltet, wodurch der Voraufwand für eine Destillation der aus der Synthese stammenden Acrylsäure geringer wird. Damit sinkt insbesondere die thermische Belastung der Acrylsäure, die zu unerwünschten Polymerisation bzw. zu einer vorzeitigen Bildung von Acrylsäure daraus resultierenden unerwünschten Polymerisation führen kann.

In einer Abwandlung der Erfindung, insbesondere in Kombination mit der erfindungsgemäßen Vorrichtung zur Herstellung von Acrylsäure mittels einer Schichtkristallisation oder einer Suspensionskristallisation, beinhaltet eine erfindungsgemäße Vorrichtung zur Herstellung von Acrylsäure folgende, miteinander fluidleitend verbundene Einheiten:
eine Synthetisierungseinheit von 3-Hydroxypropionsäure aus einem biologischen Material unter Erhalt einer 3-Hydroxypropionsäure beinhaltenden Fluiden, insbesondere wässrigen, Phase,
eine Dehydratisierungsstufe der 3-Hydroxypropionsäure unter Erhalt einer Acrylsäure beinhaltenden Fluiden, insbesondere wässrigen, Lösung und mindestens eine der folgenden Trennvorrichtungen (T1) bis (T5) als Entwässerungseinheit:
   (T1) Umkehrosmosevorrichtung,
   (T2) Elektroosmosevorrichtung,
   (T3) azeotrope Destillationsvorrichtung,
   (T4) Elektrodialysevorrichtung,
   (T5) Mehrphasentrennvorrichtung.

Diese Abwandlung kann insbesondere als Vorstufe vor der Aufreinigungseinheit eingesetzt werden. Insbesondere bevorzugt ist auch, mindestens eine der Trennvorrichtungen (T1) bis (T5) zwischen die Verfahrenschritten (a1) und (a3), insbesondere zwischen (a1) und (a2) zu schalten, um einen Vorreinigungseffekt zu erzielen.

Die Umkehrosmosevorrichtung ist geeignet zur Durchführung einer Umkehrosmose; die Elektroosmosevorrichtung ist geeignet zur Durchführung einer Elektroosmose, die azeotrope Destillationsvorrichtung ist geeignet zur Durchführung einer azeotropen Destillation; die Elektrodialysevorrichtung ist geeignet zur Durchführung einer Elektrodialyse; die Mehrphasentrennvorrichtung ist geeignet zur Durchführung einer Mehrphasentrennung, insbesondere des zuvor erwähnten "Salt-Splittings".

In einer speziellen Weiterbildung beinhaltet die Vorrichtung eine weitere Trennvorrichtung, insbesondere Filter, zum Abtrennen von Feststoffen, insbesondere Partikeln wie Mikroorganismen, Zellen oder Teilen davon, aus der fluiden Phase, aus der fluiden Lösung oder aus beiden. Die weitere Trennvorrichtung zum Abtrennen von Feststoffen befindet sich, wie vorstehend bereits beschrieben, vorzugsweise zwischen der Synthetisierungseinheit und der Dehydratisierungsstufe. Die separate Trennvorrichtung zum Abtrennen von Feststoffen kann auch zwischen der Dehydratisierungsstufe und der Aufreinigungseinheit bzw. der Trennvorrichtungen (T1) bis (T5) angeordnet sein.

Vorteilhafterweise beinhaltet die Vorrichtung weiterhin ein Protonierungsmittel der Hydroxypropionsäure in der fluiden Phase. Die Protonierung kann insbesondere durch Zugabe einer Säure, beispielsweise von Salzsäure oder Ammoniumchlorid erfolgen. Das Protonierungsmittel kann auch ein Ionenaustauscher sein, mit dem die Hydroxypropionsäure in die freie Säure überführt wird.

Das Protonierungsmittel ist insbesondere stromabwärts von der Synthetisierungseinheit, d.h. insbesondere zwischen der Synthetisierungsstufe und der Dehydratisierungsstufe oder zwischen der Dehydratisierungsstufe und einer der als Entwässerungseinheit fungierenden Trennvorrichtungen (T1) bis (T5) bzw. der Aufreinigungseinheit angeordnet. Das Protonierungsmittel kann auch direkt an der Dehydratisierungsstufe bzw. Aufreinigungseinheit bzw. Trennvorrichtung (T1) bis (T5) angeordnet sein und kann ein Zulauf für ein die Protonierung der Hydroxypropionsäure bewirkendes Reagenz, wie z.B. eine Säure, sein.

Die Vorrichtung kann ein Adsorptionsmittel oder ein Absorptionsmittel, insbesondere ein Aktivkohlefilter zur weiteren Reinigung der fluiden Phase, oder der fluiden Lösung, oder beidem, aufweisen. Mit Hilfe des Ad- bzw. Absorptionsmittels können feine, in der Suspension befindliche Partikel, wie z.B. biologische Rückstände, aus den fluiden Phase bzw. Lösungen entfernt werden, wobei dieses Absorptionsmittel sowohl zwischen der Synthetisierungseinheit und der Dehydratisierungsstufe als auch zwischen der Dehydratisierungsstufe und der Aufreinigungseinheit lokalisiert sein kann.

Die Vorrichtung zur Herstellung von Polyacrylaten beinhaltet die erfindungsgemäße Vorrichtung zur Herstellung von Acrylsäure und einen Polymerisationsreaktor zum Polymerisieren von Acrylsäure, insbesondere ein Polymerisationsreaktor zum radikalischen Polymerisieren von Acrylsäure.

Bei einer bevorzugten Ausgestaltung des erfindungsgemäßen Verfahrens zur Herstellung von Acrylsäure wird die vorstehend beschriebene Vorrichtung eingesetzt.

Bei dem Verfahren zur Herstellung von Polyacrylaten wird eine durch das erfindungsgemäße Verfahren erhaltene Acrylsäure polymerisiert, wobei diese Polymerisation vorzugsweise in Gegenwart von Vernetzern erfolgt. Wenn es sich bei den Polymeren um vernetzte, teilneutralisierte Polyacrylate handelt (sogenannte Superabsorber), so wird hinsichtlich der genauen Vorgehensweise auf das 3. Kapitel (Seite 69ff) in "Modern Superabsorbent Polymer Technology", F. L. Buchholz und A. T. Graham (Herausgeber) in, Wiley-VCH, New York, 1998 verwiesen.

Die Polyacrylate sind vorzugsweise durch einen Nachhaltigkeitsfaktor von mindestens 10, vorzugsweise mindestens 20, besonders bevorzugt mindestens 50, darüber hinaus bevorzugt mindestens 75, ferner bevorzugt mindestens 85 und weiterhin bevorzugt mindestens 95 gekennzeichnet. Der Nachhaltigkeitsfaktor gibt dabei an, zu welchem Anteil das wasserabsorbierende Polymergebilde auf nichtfossilem, nachwachsendem organischen Material basiert. Bei einem Nachhaltigkeitsfaktor von 100 besteht das Polymergebilde vollständig aus auf nichtfossilen, nachwachsendem organischen Materialen basierenden Stoffen.

Bei den durch das Verfahren zur Herstellung von Polyacrylaten erhaltenen Polyacrylaten handelt es sich vorzugsweise um ein wasserabsorbierendes Polymergebilde. Dieses wasserabsorbierende Polymergebilde weist vorzugsweise mindestens eine der folgenden Eigenschaften auf:
(β1) einen gemäß ERT 441.2-02 (ERT = Edana Recommended Test Method) bestimmten CRC-Wert (CRC = Centrifugation Retention Capacity) von mindestens 20 g/g, vorzugsweise mindestens 25 g/g und am meisten bevorzugt mindestens 30 g/g, wobei ein CRC-Wert von 60 g/g, vorzugsweise von 50 g/g nicht überschritten wird;
(β2) eine gemäß ERT 442.2-02 bestimmte Absorption unter einem Druck von 0,7 psi (AAP) von mindestens 16 g/g, vorzugsweise mindestens 18 g/g und am meisten bevorzugt mindestens 20 g/g, wobei ein Wert von 50 g/g, vorzugsweise von 40 g/g nicht überschritten wird.

Die Fasern, Folien, Formassen, Textil- und Lederhilfsmittel, Flockungsmittel, Beschichtungen oder Lacke können basieren auf Acrylsäure, welche durch das erfindungsgemäße Verfahren erhalten wurde, oder auf deren Derivaten, wie beispielsweise auf Estern dieser Acrylsäure, während gemäß der Verwendung der durch das erfindungsgemäße Verfahren zur Herstellung von Acrylsäure erhaltenen Acrylsäure diese oder ihre Derivate vorzugsweise in Fasern, Folien, Formassen, Textil- und Lederhilfsmitteln, Flockungsmitteln, Beschichtungen oder Lacken eingesetzt wird bzw. werden.

Vorteilhafte Einzelheiten und Ausgestaltungen, die jeweils angewandt oder beliebig miteinander kombiniert werden können, werden anhand der folgenden Zeichnungen, welche die Erfindung nicht einschränken, sondern lediglich exemplarisch illustrieren soll, weiter erläutert:

### Es zeigen schematisch:

- Fig. 1: ein erfindungsgemäßes Verfahrensschema;
- Fig. 2: eine bevorzugte Ausführungsform des erfindungsgemäßen Verfahrensschemas, bei der die Dehydratisierung mittels Reaktivdestillation erfolgt;
- Fig. 3: eine weitere bevorzugte Ausführungsform des erfindungsgemäßen Verfahrensschemas, bei der die Aufreinigung in einer dreistufigen Kristallisation erfolgt;
- Fig. 4 bis 6: Graphen zur Bestimmung der eutektischen Punkte.

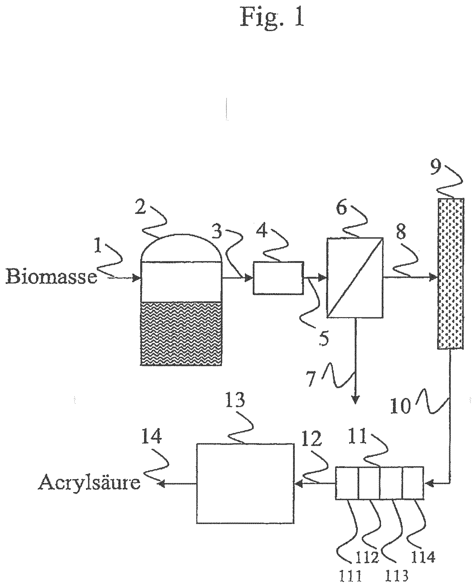

Figur 1 veranschaulicht das erfindungsgemäße Verfahrensschema sowie die erfindungsgemäße Vorrichtung. Über den Zulauf 1 für Biomasse, wie z.B. Raps oder Mais, kann diese in die Synthetisierungseinheit 2 eingebracht werden, wobei es sich bei der Synthetisierungseinheit 2 vorzugsweise um einen Bioreaktor handelt. In der Synthetisierungseinheit 2 wird die Biomasse unter Bildung einer Hydroxypropionsäuren, vorzugsweise von 2- oder 3-Hydroxypropionsäuren, am meisten bevorzugt von 3-Hydroxypropionsäure, beinhaltenden wässrigen Phase P1 umgesetzt. Über einen Ablauf 3 kann diese wässrige Phase P1 dann einer Einheit zum Abtöten, bei der es sich beispielsweise um einen Autoklaven oder eine andere, dem Fachmann bekannte Sterilisierungsvorrichtung handelt, zugeführt werden. Die so behandelte wässrige Phase P1 kann dann über den Ablauf 5 einer Filtrationseinheit 6 zugeführt werden, in der die wässrige Phase P1 von Feststoffen, wie beispielsweise Mikroorganismen, befreit wird. Über eine Abführung 7 für Feststoffe können die abgetrennten Feststoffe entfernt werden. Sofern die in der wässrigen Phase P1 enthaltenen Mikroorganismen nicht abgetötet und für einen weiteren Synthesezyklus verwendet werden sollen, können die in der Filtrationseinheit 6 abgetrennten Feststoffe zumindest teilweise auch in die Synthetisierungseinheit zurückgeführt werden (in der Fig. 1 nicht gezeigt). (die Einheiten 111, 112, 113 und 114 können auch einzeln oder in Kombination zwischen der Synthetisierungseinheit 2 und der Dehydratisierungsstufe 9 angeordnet sein, in der Figur 1 nicht gezeigt).

Über den Ablauf 8 gelangt die von Feststoffen befreite, wässrige Phase P1 dann in die Dehydratisierungsstufe 9, in der die Hydroxypropionsäure unter Abspaltung von Wasser zur Acrylsäure umgesetzt wird. Die Acrylsäure und gegebenenfalls nicht umgesetzte Hydroxypropionsäure beinhaltende wässrige Phase P2 kann dann über den Ablauf 10 in weiteren Vorrichtungsbestandteilen weiter behandelt werden. Bei diesen weiteren Vorrichtungsbestandteilen kann es sich beispielsweise um eine weitere Filtrationseinheit 111, ein Protonierungsmittel 112, ein Adsorptionsmittel 113 oder eine Entwässerungseinheit 114, beispielsweise eine Umkehrosmosevorrichtung, eine Elektroosmosevorrichtung, eine azeotrope Destillationsvorrichtung, eine Elektrodialysevorrichtung oder eine Mehrphasentrennvorrichtung, handeln. Diese Vorrichtungseinheiten können einzeln oder in Kombination hintereinander zwischen der Dehydratisierungsstufe 9 und der Aufreinigungseinheit 13 angeordnet sein. Weiterhin kann es vorteilhaft sein, eine oder mehrerer dieser weiteren Vorrichtungsbestandteile 11 anstelle oder zusätzlich zu ihrer Anordnung zwischen der Dehydratisierungsstufe 9 und der Aufreinigungseinheit 13 zwischen der Synthetisierungseinheit 2 und der Dehydratisierungsstufe 9, besonders bevorzugt zwischen der Filtrationseinheit 6 und der Dehydratisierungsstufe 9, anzuordnen (in der Figur 1 nicht gezeigt).

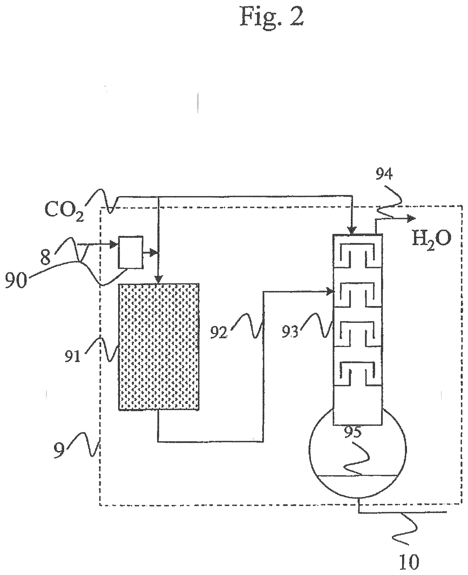

Figur 2 veranschaulicht eine bevorzugte Ausführungsform des erfindungsgemäßen Verfahrensschemas sowie der erfindungsgemäßen Vorrichtung, bei der die Dehydratisierung mittels Reaktivdestillation erfolgt. Über den Ablauf 8, über den die in der Filtrationseinheit 6 von Feststoffen befreite wässrige Phase P1 entnommen wird, gelangt diese wässrige Phase P1 meist über eine Membran 90 in einen ersten Reaktor 91, welcher einen die Dehydratisierung fördernden Katalysator enthält und in dem zumindest ein Teil der Hydroxypropionsäure zur Acrylsäure umgesetzt wird. Über eine Ableitung 92 wird dann eine wässrige Phase P2 beinhaltend Acrylsäure, Wasser, nicht umgesetzte Hydroxypropionsäure sowie gegebenenfalls weitere Nebenprodukte in einen zweiten Reaktor 93 überführt, in dem es sich beispielsweise um einen, ebenfalls einen die Dehydratisierung fördernden Katalysator beinhaltende Destillations- oder Rektifikationskolonne handelt, überführt. In diesem zweiten Reaktor 93 wird nun die noch nicht umgesetzte Hydroxypropionsäure weiter zur Acrylsäure umgesetzt und zugleich das in der wässrigen Phase enthaltene Wasser über die Abführung 94 abgetrennt (Reaktivdestillation). Das im zweiten Reaktor 93 erhaltene Sumpfprodukt 95, welches die Acrylsäure beinhaltet, kann dann über die Ableitung 10 den weiteren Vorrichtungsbestandteilen 11 oder aber unmittelbar der Aufreinigungseinheit 14 zugeführt werden (siehe Figur 1). Über eine Zuleitung 96 können sowohl der erste Reaktor als auch der zweite Reaktor mit Kohlendioxid beaufschlagt werden, um Decarboxylierungsreaktionen während der Dehydratisierung zu verhindern.

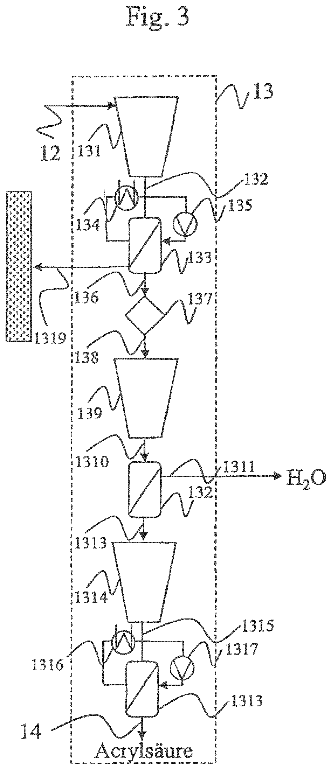

Fig. 3 zeigt ebenfalls eine bevorzugte Ausführungsform des erfindungsgemäßen Verfahrensschemas, wobei bei dieser Ausführungsform die Aufreinigung in einer dreistufigen Kristallisation erfolgt. Über den Zulauf 12 gelangt die in den weiteren Vorrichtungsbestandteilen 11 behandelte wässrige Phase P2 oder aber die unmittelbar aus der Dehydratisierungsstufe 9 erhaltene wässrige Phase P2 in eine erste Kristallisationsvorrichtung 131, bei der es sich vorzugsweise um einen Suspensionskristallisator handelt. Denkbar ist jedoch auch der Einsatz andere Kristallisationsvorrichtungen, wie etwa eines Schichtkristallisators. In der ersten Kristallisationsvorrichtung 131 wird die Acrylsäure beinhaltende wässrige Phase P2 vorzugsweise soweit abgekühlt, dass Wasser und Acrylsäure auskristallisieren. Sofern als erste Kristallisationsvorrichtung ein Suspensionskristallisator eingesetzt wird, wird die so erhaltene Kristallsuspension über die Ableitung 132 einem ersten Trennbereich 133 zugeführt, bei dem es sich vorzugsweise um eine Waschkolonne handelt und in der die Kristalle von der zurückbleibenden Mutterlauge abgetrennt werden. Sofern als erste Kristallisationsvorrichtung ein Schichtkristallisator eingesetzt wird, ist der Einsatz einer Trenneinheit entbehrlich (in Figur 3 nicht gezeigt), da in diesem Fall das Abtrennen der Kristalle von der Mutterlauge beispielsweise mittels einer Waschkolonne aufgrund der immobilisierung der Kristalle an den Oberflächen des Schichtkristallisators nicht erforderlich ist. Die in der ersten Trenneinheit von der Mutterlauge abgetrennten Kristalle können zum Zwecke der weiteren Reinheitssteigerung mittels eines Aufschmelzers 134 zumindest teilweise aufgeschmolzen und die aufgeschmolzenen Kristalle können mittels eines Förderelementes 135, beispielsweise mittels einer Pumpe, im Gegenstrom zur Kristallwäsche eingesetzt werden, so wie dies in der DE-A-101 49 353 beschrieben ist. Wurde als erste Kristallisationsvorrichtung 131 ein Schichtkristallisator eingesetzt, so kann es auch hier vorteilhaft sein, die Kristalle mindestens teilweise aufzuschmelzen und die erhaltene Kristallschmelze im Gegenstrom zur Kristallwäschen einzusetzen.

Die im ersten Trennbereich nach dem Abtrennen der Mutterlauge erhaltenen Kristalle, welche im Wesentlichen aus Wasser und Acrylsäure bestehen, können dann über die Ableitung 136 einem Aufschmelzer 137 zugeführt werden, in dem die Kristalle möglichst vollständig aufgeschmolzen werden. Die zurückbleibende Mutterlauge, welche noch größere Mengen an nicht umgesetzter Hydroxypropionsäure enthalten kann, kann vorteilhafterweise über die Ableitung 1319 in die Dehydratisierungsstufe 9 zurückgeführt werden. Die so aufgeschmolzenen Kristalle werden dann über die Ableitung 138 einer zweiten Kristallisationsvorrichtung 139 zugeführt, in der die Temperatur soweit abgesenkt wird, dass sich im Wesentlichen aus Wasser bestehende Kristalle bilden. Bei dieser zweiten Kristallisationsvorrichtung handelt es sich ebenfalls vorzugsweise um einen Suspensionskristallisator, jedoch ist auch hier der Einsatz eines Schichtkristallisators denkbar. Mittels der Ableitung 1310 kann, sofern als zweite Kristallisationsvorrichtung 139 ein Suspensionskristallisator eingesetzt wurde, die in der zweiten Kristallisationsvorrichtung 139 erhaltene Kristallsuspension dann einem zweiten Trennbereich 132 zugeführt werden, in dem die im Wesentlichen auf Wasser basierenden Kristalle über eine Abführung 1311 von der im Wesentlichen auf Acrylsäure und Wasser basierenden Mutterlauge abgetrennt werden. Auch hier ist im Falle eines Einsatzes eines Schichtkristallisators als Kristallisationsvorrichtung die Verwendung einer getrennten Trenneinheit, wie beispielsweise einer Waschkolonne, entbehrlich.

Die im zweiten Trennbereich 132 von den Kristallen abgetrennte Mutterlauge kann dann über die Ableitung 1313 der dritten Kristallisationsvorrichtung 1314 zugeführt werden, in der die Temperatur soweit abgesenkt wird, dass im Wesentlichen auf Acrylsäure basierende Kristalle gebildet werden. Bei dieser dritten Kristallisationsvorrichtung 1314 kann es sich ebenfalls um eine Suspensionskristallisator oder um einen Schichtkristallisator handeln. Sofern in der dritten Kristallisationsvorrichtung ein Suspensionskristallisator eingesetzt wurde, kann die in der dritten Kristallisationsvorrichtung erhaltene Kristallsuspension über die Ableitung 1315 dem dritten Trennbereich 1316 zugeführt werden, in dem die Acrylsäurekristalle von der Mutterlauge angetrennt werden. Wie auch bei der ersten Kristallisationsstufe kann es vorteilhaft sein, zumindest einen Teil der Kristalle im dritten Trennbereich mittels eines Aufschmelzers 1317 aufzuschmelzen und dann über ein Förderelement 1318, wie etwa einer Pumpe, im Gegenstrom zur Kristallwäsche einzusetzen. Die im dritten Trennbereich 1316 erhaltenen Acrylsäurekristalle können dann über eine Ableitung 14 entnommen werden. Wurde als dritte Kristallisationsvorrichtung 1314 ein Schichtkristallisator eingesetzt, so können auch hier die Kristalle zumindest teilweise aufgeschmolzen werden und die geschmolzenen Kristalle zur Kristallwäsche eingesetzt werden.

Die vorliegende Erfindung wird nun anhand nichtlimitierender Beispiele näher erläutert.

### BEISPIELE

### Herstellungsbeispiel 1

Herstellen einer eine Fermentationsbrühe simulierenden Testzusammensetzung 1 beinhaltend:
1.000 ml Wasser
100 g 3-Hydroxypropionsäure (10 Gew.-%)
9 g Bäckerhefe
1 g Glukose als organisches Material,
wobei mittels Ammoniak ein pH-Wert von 6,5 bis 7,5 eingestellt wurde.

### Herstellungsbeispiel 2

Herstellen einer eine gereinigte Fermentationsbrühe simulierenden Testzusammensetzung 2 beinhaltend:
1.000 ml Wasser
100 g 3-Hydroxypropionsäure (10 Gew.-%)

### DEHYDRATISIERUNG 1:

Die im Herstellungsbeispiel 1 erhaltene Lösung wird in einer in Fig. 2 gezeigten Vorrichtung zunächst durch eine Membran 90 (kommerziell erhältlich bei Amafilter GmbH) von der Hefe befreit und in einem ersten Druckreaktor 91 (durchströmtes Edelstahlreaktorrohr mit einem Innendurchmesser von 17 mm und einer Länge von 50 cm) in Gegenwart von Phosphorsäure in einer Konzentration von 1 Teil Phosphorsäure auf 5.000 Teile Eduktstrom über 8 Minuten Verweilzeit bei einer Temperatur von 140°C und einem Druck von 8 bar erhitzt.

Die so erhaltene Reaktionsmischung, welche vor allem Wasser, nicht umgesetzte 3-Hydroxypropionsäure und Acrylsäure beinhaltet, wurde anschließend in eine Reaktionskolonne 93 gegebenen (Autoklav mit aufgesetzter Destillationskollonne) und ebenfalls in Gegenwart der anfangs eingesetzten Phosphorsäure als Katalysator bei einer Temperatur von 140°C und einem Druck von 3,7 bar über 16 Minuten Verweilzeit erhitzt. Dabei wurde das in der Reaktionsmischung enthaltene Wasser über Kopf abgetrennt und ein Acrylsäure-reiches Sumpfprodukt erhalten.

### DEHYDRATISIERUNG 2:

Hierbei wurde die Dehydratisierung 1 mit folgenden Unterschieden wiederholt: Die aus dem Druckreaktor 91 erhaltene Reaktionsmischung, welche vor allem Wasser, nicht umgesetzte 3-Hydroxypropionsäure und Acrylsäure beinhaltet, wurde anschließend in eine Reaktionskolonne 93 gegebenen (Autoklav mit aufgesetzter Destillationskollonne) und ebenfalls in Gegenwart der anfangs eingesetzten Phosphorsäure als Katalysator bei einer Temperatur von 120°C und einem Druck von 500 mbar über 45 Minuten Verweilzeit erhitzt. Dabei wurde das in der Reaktionsmischung enthaltene Wasser über Kopf abgetrennt und ein Acrylsäure-reiches Sumpfprodukt erhalten.

### DEHYDRATISIERUNG 3:

Hierbei wurde die Dehydratisierung 1 mit folgenden Unterschieden wiederholt: Anstelle von Phosphorsäure wurde CO₂ in den Reaktor 91 bis zur Sättigung eingespeist.

### DEHYDRATISIERUNG 4:

Hierbei wurde die Dehydratisierung 1 mit dem Unterschieden wiederholt, dass Testzusammensetzung 2 eingesetzt wurde.

### DEHYDRATISIERUNG 5:

Hierbei wurde die Dehydratisierung 2 mit dem Unterschieden wiederholt, dass Testzusammensetzung 2 eingesetzt wurde.

### DEHYDRATISIERUNG 6:

Hierbei wurde die Dehydratisierung 3 mit dem Unterschieden wiederholt, dass Testzusammensetzung 2 eingesetzt wurde.

Die Sumpfprodukte der vorstehenden Dehydratisierungen haben die in Tabelle 1 dargestellten Zusammensetzungen.

**Tabelle 1**

| **Dehydratisierung** | **1** | **2** | **3** |
|---|---|---|---|
| **Wasser** | 3 % | 4 % | 4 % |
| **3-Hydroxypropionsäure** | 11 % | 15 % | 18 % |
| **Acrylsäure** | 85 % | 80 % | 77 % |
| **Glukose** | 1 % | 1 % | 1 % |
| **Dehydratisierung** | 4 | 5 | 6 |
| **Wasser** | 6 | 5 % | 6 % |
| **3-Hydroxypropionsäure** | 9 % | 15 % | 18 % |
| **Acrylsäure** | 87 % | 79 % | 76 % |

### AUFARBEITUNG 1

Das acrylsäurereiche Sumpfprodukt der Dehydratisierung 1 wurde einem Suspensionskristaller (Typ MSMPR "Mixed Suspension Mixed Product Removal" wie in Arkenbout G.F. "Melt Crystallization Technology", Lancaster, Technomic Publishing Company Inc. 1995 beschrieben) mit einem Volumen von 1L zugeführt und auf eine Gleichgewichtstemperatur von 0°C mit einer Temperaturrampe von 0,25K/min heruntergekühlt. Die erhaltene Kristallsuspension, mit einem Feststoffgehalt von ca. 50 Gew.-%, wurde über eine gewöhnliche Labornutsche in Kristalle und verunreinigte Mutterlauge getrennt. Im Anschluss wurden die Kristalle mit gereinigter Acrylsäure (99,5 Gew.-%) in einem Massenverhältnis (Kristalle / Reinigungsflüssigkeit) von 5:1 gewaschen und von anhaftenden Verunreinigungen befreit. Die gereinigten Kristalle wurden auf Raumtemperatur gebracht und dabei geschmolzen. Hierbei wurde eine Acrylsäure mit einer Reinheit von 99,5 Gew.-% gewonnen. Die Zusammensetzungen der Phasen (Feed, Kristalle und Mutterlauge) sind in Tabelle 2 angegeben.

**Tabelle 2**

| | **Feed** | **Kristallphase** | **Mutterlauge** |
|---|---|---|---|
| **Wasser** | 3 % | 0,1 % | 5,9 % |
| **3-Hydroxypropionsäure** | 11 % | 0,37 % | 21,63 % |
| **Acrylsäure** | 85 % | 99,5 % | 70,5 % |
| **Glukose** | 1 % | 0,03 % | 1,97 % |

### DEHYDRATISIERUNG 7

Dehydratisierung 4 wurde mit dem Unterschied wiederholt, dass direkt nach Druckreaktor 91 das Dehydratisierungsprodukt entspannt wurde und die in Feed 1 Tabelle 3 angegebene Zusammensetzung aufweist.

### AUFARBEITUNG 2

### Stufe A)

Die wasserreiche Acrylsäure-Mischung des Feeds 1 wurde in einen 1L-Rührwerkskristaller eingefüllt und mit einer Temperaturrampe von 0,25 K/min bis auf einen Temperatur von -18°C nahe an den Tripeleutektischen Punkt heruntergekühlt. Die Suspension wurde anschließend über eine gewöhnliche Labornutsche filtriert. Die erhaltenen Kristalle wurden auf Raumtemperatur erwärmt und geschmolzen. Die Zusammensetzung der Kristalle 1 und der Mutterlauge 1 sind in Tabelle 3 aufgeführt. Die Kristallisation wurde mehrmals analog durchgeführt, um eine entsprechende Produktmenge für die nachfolgenden Kristallisationsschritte zu erhalten.

### Stufe B)

Die vorwiegende aus Wasser und Acrylsäure bestehende Kristallphase 1 der Stufe A wurden wiederum in einen 1L-Rührwerkskristaller eingefüllt und mit einer Temperaturrampe von 0,25 K/min auf eine Temperatur von -10°C heruntergekühlt. Die Kristallisation dieses quasi binären Systems aus Wasser und Acrylsäure wurde vor Erreichen des binären Eutektikums abgebrochen, so dass gezielt im wesentlichen Wasser kristallisiert. Die erhaltene Suspension wurde im Anschluss aus dem Kristaller abgelassen und über eine Laborfilternutsche in Kristalle und Mutterlauge getrennt. Die Zusammensetzungen der Mutterlauge 2 und der Kristallphase 2 sind ebenfalls in Tabelle 3 aufgeführt. Diese Kristallisation wurde ebenfalls mehrmals durchgeführt, um eine entsprechende Einsatzmenge für den nachfolgenden Kristallisationsschritt bereitzustellen.

### Stufe C)

Die Mutterlauge 2 der Stufe B mit einer quasi eutektischen Zusammensetzung von Wasser und Acrylsäure wurde in einen 1L- Rührwerkskristaller eingefüllt und mit einer Temperaturrampe von 0,25 K/min auf ca. -15°C unterhalb des bineren euetektischen Punktes heruntergekühlt. Die hierbei entstandene Suspension aus Wasser- und Acrylsäure-Kristallen wurde über eine Labornutsche gegeben und filtriert. Die Zusammensetzung der Mutterlauge 3 und der Kristallphase 3 sind in Tabelle 3 ebenfalls aufgeführt.

**Tabelle 3**

| **Stufe A)** | **Feed 1** | **Kristallphase 1** | **Mutterlauge 1** |
|---|---|---|---|
| **Wasser** | 80,0% | 79,0% | 80,4% |
| **3-Hydroxypropionsäure** | 5,0% | 1,0% | 6,7% |
| **Acrylsäure** | 15,0% | 20,0% | 12,9% |
| **Stufe B)** | **Feed 2 = Kristallphase 1** | **Kristallphase 2** | **Mutterlauge 2** |
| **Wasser** | 79,0% | 99,5% | 35,2% |
| **3-Hydroxypropionsäure** | 1,0% | 0,02% | 3,1% |
| **Acrylsäure** | 20,0% | 0,48% | 61,7% |

| **Stufe C)** | **Feed 3 = Mutterlauge 2** | **Kristallphase 3** | **Mutterlauge 3** |
|---|---|---|---|
| **Wasser** | 35,2% | 38,0% | 30,1% |
| **3-Hydroxypropionsäure** | 3,1% | 0,00% | 9,6% |
| **Acrylsäure** | 61,7% | 62,00% | 66,4% |

Dadurch wurde eine Acrylsäure-Wasser-Phase mit einem Acrylsäuregehalt von 62% und einem Wassergehalt von 38% erhalten.

Die aus der Kristallisation erhaltene Monomerlösung bestehend aus 260 g Acrylsäure (62%), die zu 70 Mol-% mit Natronlauge neutralisiert wurde (202,054 g 50%ige NaOH), 160 g Wasser (38%), 0,409 g Polyethylenglykol-300-diacrylat, 1,253 g Monoallylpolyethylenglykol-450-monoacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,3 g Natriumperoxydisulfat in 10 g H₂O, 0,07 g 35%ge WasserstoffperoxidLösung in 10 g H₂O und 0,015 g Ascorbinsäure in 2 g H₂O) zugesetzt. Nachdem die Endtemperatur von ca.100°C erreicht war, wurde das entstandene Gel mit einem Fleischwolf zerkleinert und bei 150°C 2 Stunden lang im Trockenschrank getrocknet. Das getrocknete Polymerisat wurde grob zerstoßen, mittels einer Schneidmühle SM 100 mit einem 2 mm-Konidurlochung gemahlen und als Pulver A auf eine Partikelgröße von 150 bis 850 µm gesiebt.

100 g des Pulvers A werden mit einer Lösung bestehend aus 1,0 g Ethylencarbonat (EC), 0,6 g Al₂(SO₄)₃×14 H₂O und 3 g entionisiertes Wasser vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver A in einem Umluftschrank bei 185°C für 30 Minuten erhitzt. Es wurde ein Pulver B erhalten (Korngrößen: auf 150 µm Maschenweite 13 %, auf 300 µm Maschenweite 15 %, auf 400 µm Maschenweite 12 %, auf 500 µm Maschenweite 15 %, auf 600 µm Maschenweite 20 %, auf 710 bis 850 µm Maschenweite 25 %). Die Eigenschaften dieses Pulvers sind in Tabelle 4 angegeben.

**Tabelle 4**

| Pulver | Beschichtung mit | | Eigenschaften | |
|---|---|---|---|---|
| | EC [Gew.-%] | Al-sulfat [Gew.-%] | CRC [g/g] | AAP_{(0.7} psi) [g/g] |
| A | 0 | 0 | 33,8 | 20* |
| B | 1,0 | 0,6 | 29,6 | 23,7 |
| * bei 0,3 psi bestimmt. | | | | |

### TESTMETHODEN

### SCHMELZPUNKT-BESTIMMUNG ÜBER DSC

### Gerät:

DSC 820 von Mettler Toledo mit Keramiksensor FRS5 und Silberofen (-150 bis 700 °C)

### Prinzip:

Man bestimmt die Temperatur (den Temperaturbereich) der Phasenumwandlung vom festen in den flüssigen Zustand. In der Praxis wird eine Probe der zu untersuchenden Substanz bei Atmosphärendruck erhitzt und dabei werden die Temperaturen des Schmelzbeginns sowie des vollständigen Schmelzens bestimmt. Als Methode wird die Dynamische Differenzkalorimetrie (DDK = DSC) eingesetzt. Nach DIN 51 005 versteht man darunter eine thermoanalytische Methode, bei der durch die Messung der Temperaturdifferenz an einer definierten Wärmeleitstrecke zwischen Probe und Referenz, die gleichzeitig demselben Temperaturprogramm unterworfen werden, die quantitative Erfassung der Wärmestromdifferenz ermöglicht wird. Dieser Wärmestrom wird als Funktion der Referenztemperatur gemessen und aufgezeichnet. Die Proportionalitätskonstante K, d.h. der Kalibrierfaktor, ist abhängig vom Wärmewiderstand und damit abhängig von der Temperatur. Sie muss experimentell bestimmt werden. Schmelzen ist mit einer endothermen Enthalpieänderung verbunden, Kristallisieren (Gefrieren, Erstarren) mit einer exothermen.

### Schmelzpunkt:

Als Schmelztemperatur bezeichnet man diejenige Temperatur, bei der unter atmosphärischem Druck der Übergang zwischen fester und flüssiger Phase stattfindet. Unter idealen Bedingungen entspricht diese Temperatur der Gefriertemperatur. Da bei vielen Stoffen der Phasenübergang in einem Temperaturbereich stattfindet, wird dieser Übergang auch oft als Schmelzbereich bezeichnet. Für Reinsubstanzen gibt es nur einen effektiven Schmelzpunkt, d.h. nur eine einzige Temperatur, bei der Feststoff und Flüssigkeit im Gleichgewicht stehen. Alle Verunreinigungen und Beimischungen von anderen Komponenten, oder z.B. eine zweite oder dritte Hauptkomoponente, ergeben einen Schmelzbereich, in dem beide Phasen, Schmelze und Feststoff, über einen gewissen Temperaturbereich mit unterschiedlichen Zusammensetzungen gleichzeitig auftreten. Hier wird mit der extrapolierten Onset-Temperatur der Beginn des Schmelzens angezeigt. Der Schmelzbereich gibt das effiktive Zweiphasengebiet an, in dem Schmelze und Feststoff im thermodynamischen Gleichgewicht stehen. Oft genügt es nicht, das Schmelzen einer Substanz nur mit dem Schmelzpunkt zu beschreiben. Die Aufzeichnung des gesamten Schmelzverlaufs ist vor allem dann angezeigt, wenn die Substanz nicht rein, sondern eine Mischung aus mehreren ähnlichen Stoffen, anderen Stoffen oder Modifikationen (Polymorphie) ist, und eventuell gleichzeitige Zersetzungen aufweist. Auch Kunststoffe mit breitem Kristallitschmelzbereich erfordern die Bestimmung des gesamten Schmelzverhaltens.

### Auswertung:

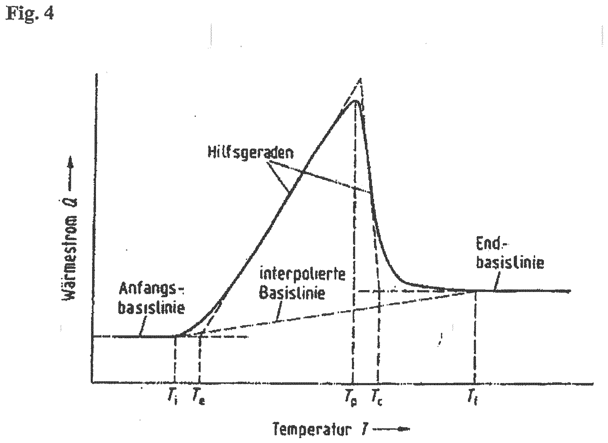

Die DSC-Messdaten werden in einer Kurve dargestellt, in der die Wärmestromdifferenz oder die dieser zugeordnete Temperaturdifferenz gegen die Temperatur oder die Zeit aufgetragen ist. Dabei werden Wärmestromdifferenzen von endothermen Vorgängen in positiver und solche von exothermen Vorgängen in negativer Ordinatenrichtung aufgetragen (siehe DIN 51007). Zur Charakterisierung eines Schmelzpeaks können nach DIN 51004 folgende Temperaturen herangezogen werden (siehe Fig. 4): Peakanfangstemperatur, extrapolierte Peakanfangstemperatur, Peakmaximumtemperatur, extrapolierte Peakendtemperatur sowie Peakendtemperatur (Begriffe siehe DIN 51005). Die extrapolierte Peakanfangstemperatur wird der Schmelztemperatur zugerechnet. Die Peakmaximum-Temperatur (bei exponentiellem Abfall der Peakflanke) entspricht dem Klarschmelzpunkt, d.h. dem Ende des Schmelzbereichs, bei dem die geschmolzene Substanz nicht mehr durch suspendierte Kristalle getrübt ist. Er wird auch Schmelzpunkt der letzten Kristalle oder Liquiduspunkt genannt.

### Definition charakteristischer Temperaturen eines Peaks (nach DIN 51004)

In Fig. 4 bedeuten:
Ti Peakanfangstemperatur; dort beginnt die Messkurve von der extrapolierten Anfangsbasislinie abzuweichen
Te Extrapolierte Peakanfangstemperatur; dort schneidet die Hilfsgerade durch die ansteigende Peakflanke die extrapolierte Anfangsbasislinie
Tp Peakmaximumtemperatur; dort liegt das Maximum der Differenzen zwischen Messkurve und interpolierter Basislinie (dies ist nicht unbedingt das absolute Maximum der Messkurve)
Tc Extrapolierte Peakendtemperatur; dort schneidet die Hilfsgerade durch die absteigende Peakflanke die extrapolierte Endbasislinie
Tf Peakendtemperatur; dort erreicht die Messkurve wieder die extrapolierte Endbasislinie

Die Basislinie wird in der Praxis nach verschiedenen Verfahren (siehe DIN 51007) zwischen Peakanfangs- und Peakendtemperatur interpoliert. Für die Definition der extrapolierten Peakanfangs- und Peakendtemperatur kann in den meisten Fällen mit genügender Genauigkeit eine zwischen Peakanfang und Peakende linear interpolierte Basislinie verwendet werden. Die Hilfsgeraden werden entweder als Wendetagenten oder als Ausgleichsgeraden durch den (fast) linearen Teil der beiden Peakflanken gelegt. Die Unterscheidung der beiden Verfahren hat für die (Kalibrier-)Praxis keine Bedeutung, da die sich ergebenden Unterschiede viel kleiner sind als die Wiederholstreuungen der Messungen. Die übliche Charakterisierung des Schmelzpeaks erfolgt durch die extrapolierte Peakanfangstemperatur.

### Bestimmung des Phasendiagramms eines Dreistoffgemsiches

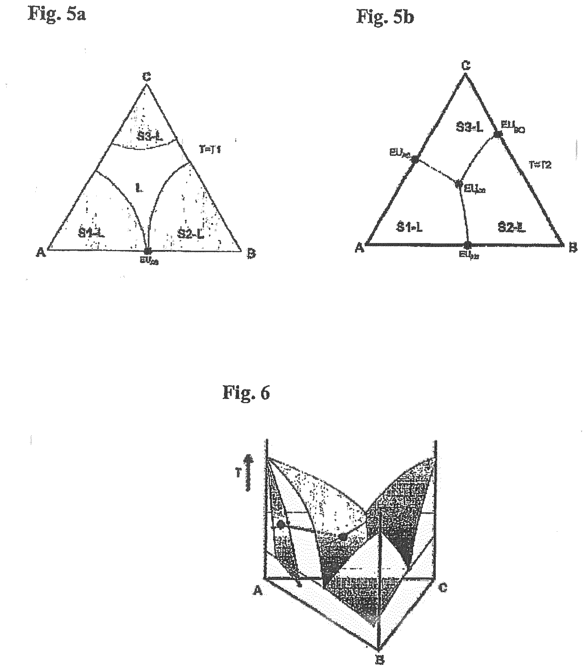

Nachdem für verschiedene Mischungen eines Dreistoffsystems die Schmelzpunkte bzw. die Schmelzbereiche, d.h. die Zweiphasenbereiche, vermessen wurden, können diese in ein Dreidimensionales Dreiecksdiagramm eingetragen werden. Basierend auf einer entsprechenden Auswertesoftware, z.B. Windows Excel, kann die Visualisierung erfolgen. Die resultierenden Flächen der vermessenen Schmelzpunkte erzeugen bei eutektischen System sog, eutektische Rinnen, die sich in einem definierten Punkt, dem sog. Triple-Eutektikum schneiden. In Fig. 5a ist ein planarer isothermer Schnitt durch ein dreidimensionales Dreiecksdiagramm dargestellt. Fig. 5b zeigt eine entsprechende Draufsicht des Phasendiagramms mit Tripleeutektikum. Eine dreidimensionale Darstellung zeigt Fig. 6.

### BESTIMMUNG DER RETENTION

Die als CRC bezeichnete Retention wird nach der ERT 441.2-02 bestimmt, wobei "ERT" für "EDANA recommended Test" und "EDANA" für European Disposables and Nonwovens Association" steht.

### BESTIMMUNG DER ABSORPTION UNTER DRUCK

Die als AAP bezeichnete Absorption gegen einen Druck von 0,7 psi wird nach der ERT 442.2-02 bestimmt.

### BESTIMMUNG DER TEILCHENGRÖßE

Die Teilchengrößen werden vorliegend nach ERT 420.2-02 bestimmt, wobei die hier angegebenen Siebe verwendet werden.

### BESTIMMUNG DER PHASENZUSAMMENSETZUNG

Die Phasenzusammensetzungen bei der Kristallisation werden durch HPLC bestimmt und in % angegeben.

### Bezugszeichenliste

- 1: Zulauf für Biomasse
- 2: Synthetisierungseinheit
- 3: Ablauf für eine Fermentationslösung
- 4: Einheit zum Abtöten
- 5: Ablauf für eine Fermentationslösung beinhaltend abgetötete Mikroorganismen
- 6: Filtrationseinheit
- 7: Abführung für abgetrennte Feststoffe
- 8: Ablauf für eine von Feststoffen befreite Fermentationslösung
- 9: Dehydratisierungsstufe
90 Membran/Filter
91 erstes Reaktionsgefäß
92 Ableitung für wässrige Phase beinhaltend zumindest teilweise umgesetzte Hydroxypropionsäure
93 als Destillationsvorrichtung ausgebildetes, zweites Reaktionsgefäß
94 Abführung für Wasser
95 Sumpfprodukt beinhaltend Wasser, nicht umgesetzte Hydroxypropionsäure und Acrylsäure
96 Zuleitung für Kohlendioxid
- 10: Ablauf für eine wässrige Phase beinhaltend Acrylsäure, Wasser und gegebenenfalls nicht umgesetzte Hydroxypropionsäure
- 11: weitere Vorrichtungsbestandteile, beispielsweise
111 weitere Filtrationseinheiten
112 Protonierungsmittel
113 Adsorptionsmittel
114 Entwässerungseinheiten
- 12: Zuleitung für eine wässrige Phase beinhaltend Acrylsäure, Wasser und gegebenenfalls nicht umgesetzte Hydroxypropionsäure
- 13: Aufreinigungseinheit
- 131: erste Kristallisationsvorrichtung
- 132: Ableitung für die in der ersten Kristallisationsvorrichtung erhaltene Kristallesuspension
- 133: erster Trennbereich
- 134: Aufschmelzer zum Aufschmelzen mindestens eines Teils der in der ersten Kristallisationsvorrichtung erhaltenen Kristalle
- 135: Förderelement, z. B. Pumpe
- 136: Ableitung die im ersten Trennbereich von der Mutterlauge abgetrennten Kristalle im Wesentlichen beinhaltend Wasser und Acrylsäure
- 137: Aufschmelzer
- 138: Ableitung der im Aufschmelzer erhaltenen Kristallschmelze
- 139: zweite Kristallisationsvorrichtung
- 1310: Ableitung für die in der zweiten Kristallisationsvorrichtung erhaltene Kristallsuspension
- 1311: Abführung für die im zweiten Trennbereich erhaltenen Kristalle im Wesentlichen beinhaltend Wasser
- 1312: zweiter Trennbereich
- 1313: Ableitung der im zweiten Trennbereich von den Kristallen abgetrennten Mutterlauge
- 1314: dritte Kristallisationsvorrichtung
- 1315: Ableitung für die in der dritten Kristallisationsvorrichtung erhaltene Kristallesuspension
- 1316: dritter Trennbereich
- 1317: Aufschmelzer zum Aufschmelzen mindestens eines Teils der in der dritten Kristallisationsvorrichtung erhaltenen Kristalle
- 1318: Förderelement, z. B. Pumpe
- 1319: Ableitung für die im ersten Trennbereich abgetrennte Mutterlauge
- 14: Ableitung für aufgereinigte Acrylsäure

## Patentansprüche

1. Verfahren zur Herstellung von Acrylsäure, beinhaltend die Verfahrenschritte:
(a1) Bereitstellen einer Hydroxypropionsäure beinhaltenden wässrigen Phase P1,
(a2) Dehydratisieren der Hydroxypropionsäure unter Erhalt einer Acrylsäure beinhaltenden wässrigen Phase P2,
(a3) Aufreinigung der Acrylsäure beinhaltenden wässrigen Phase P2, durch eine Kristallisation unter Erhalt einer gereinigten Phase,
wobei die Dehydratisierung der Hydroxypropionsäure im Verfahrensschritt (a2) durch eine Flüssigphasendehydratisierung, mittels einer Reaktivdestillation erfolgt, wobei die Dehydratisierung in mindestens zwei Reaktoren durchgeführt wird, beinhaltend die folgenden Verfahrensschritte:
- Erhitzen der wässrige Phase P1 in mindestens einen ersten Reaktor R1 in Gegenwart eines homogenen oder heterogenen Katalysators unter Erhalt eines ersten, Acrylsäure beinhaltenden wässrigen Fluids F1_1 unter einem Druck Π1;
- Einbringen dieses Fluids F1_1 in einen weiteren Reaktor R2;
- Erhitzen des in den Reaktor R2 eingebrachten Fluids F1_1 in Gegenwart eines Katalysators unter einem Druck Π2 unter Erhalt eines Fluides F1_2,
wobei Π2 und Π1 ungleich sind.

2. Verfahren nach Anspruch 1, wobei die Phase P1 erhalten wird durch ein Verfahren beinhaltend die Verfahrensschritte:
i) Herstellung von Hydroxypropionsäure aus einem biologischen Material unter Erhalt einer Hydroxypropionsäure sowie Mikroorganismen beinhaltenden, wässrigen Phase,
ii) gegebenenfalls Abtöten der Mikroorganismen,
iii) Abtrennen von Feststoffen aus der wässrigen Phase,

3. Verfahren nach einem der vorhergehenden Ansprüche, wobei die Phase P1 wässerig ist und eine Zusammensetzung umfassend
(Z1_1) 1 bis 40 Gew.-% Hydroxypropionsäure, deren Salze oder Mischungen davon,
(Z1_2) 0,1 bis 5 Gew.-% anorganische Salze,
(Z1_3) 0,1 bis 30 Gew.-% von Hydroxypropionsäure verschiedene, organische Verbindungen,
(Z1_4) 0 bis 50 Gew.-% Feststoffe, sowie
(Z1_5) 20 bis 90 Gew.-% Wasser,
wobei die Summe der Komponenten (Z1_1) bis (Z1_5) 100 Gew.-% beträgt, aufweist.

4. Verfahren nach einem der vorhergehenden Ansprüche, wobei die gereinigte Phase im Hinblick auf die Acrylsäure eine Reinheit von mindestens 40% aufweist.

5. Verfahren nach einem der vorhergehenden Ansprüche, wobei die Hydroxypropionsäure in Phase P1 oder die Acrylsäure in Phase P2 vor der Durchführung mindestens eines der Verfahrensschritte (a2) oder (a3) durch den Zusatz von Säuren in ihre protonierte Form überführt wird.

6. Verfahren nach einem der vorhergehenden Ansprüche, wobei die Hydroxypropionsäure 2-Hydroxypropionsäure ist.

7. Verfahren nach einem der Ansprüche 1 bis 5, wobei die Hydroxypropionsäure 3-Hydroxypropionsäure ist.

8. Vorrichtung zur Herstellung von Acrylsäure beinhaltend folgende fluidleitend miteinander verbundene Einheiten:
- eine Syntheseeinheit zur Herstellung von Hydroxypropionsäure, gefolgt von,
- einer Dehydratisierungsstufe zur Umwandlung der Hydroxypropionsäure in Acrylsäure, wobei die Dehydratisierungsstufe mindestens zwei fluidleitend miteinander verbundene Reaktoren R1 und R2 aufweist, von denen einer (R1) mit einem Druck Π1 und der andere (R2) mit einem Druck Π2 belastbar ist, wobei der Druck Π2 von Druck Π1 verschieden ist, gefolgt von
- Aufreinigungseinheit der Acrylsäure beinhaltenden Phase P2 durch eine Suspensionskristallisation oder eine Schichtkristallisation unter Erhalt einer gereinigten Phase,
wobei Reaktor R2 als Destillations- oder Rektifikationsvorrichtung ausgebildet ist und der Reaktor R1 nicht.

9. Vorrichtung nach Anspruch 8, wobei Aufreinigungseinheit folgende, fluidleitend miteinander verbundene Bestandteile beinhaltet:
(δ1) einen ersten Kristallisationsbereich, einen zweiten Kristallisationsbereich einen dritten Kristallisationsbereich, einen ersten Trennbereich, einen zweiten Trennbereich, einen dritten Trennbereich, mindestens einen Aufschmelzer, und mindestens sechs Führungen,
(δ2) der erste Kristallisationsbereich ist mit dem ersten Trennbereich über eine erste Führung verbunden,
(δ3) der erste Trennbereich ist mit dem mindestens einen Aufschmelzer über eine zweite Führung verbunden,
(δ4) der mindestens eine Aufschmelzer ist mit dem zweiten Kristallisationsbereich über eine dritte Führung verbunden,
(δ5) der zweite Kristallisationsbereich ist mit dem zweiten Trennbereich über eine vierte Führung verbunden,
(δ6) der zweite Trennbereich ist mit dem dritten Kristallisationsbereich über eine fünfte Führung verbunden,
(δ7) der dritte Kristallisationsbereich ist mit dem dritten Trennbereich über eine sechste Führung verbunden.

10. Verfahren zur Herstellung von Acrylsäure nach einem der Ansprüche 1 bis 7, wobei eine Vorrichtung nach Anspruch 8 oder 9 eingesetzt wird.

## Claims

1. Process for producing acrylic acid, comprising the process steps:
(a1) preparing an aqueous phase P1 comprising hydroxypropionic acid,
(a2) dehydrating the hydroxypropionic acid, obtaining an aqueous phase P2 comprising acrylic acid,
(a3) purifying the aqueous phase P2 comprising acrylic acid, by crystallization, obtaining a purified phase,
wherein the dehydration of hydroxypropionic acid in process step (a2) takes place by liquid-phase dehydration, by means of a reactive distillation, wherein the dehydration is carried out in at least two reactors, comprising the following process steps:
- heating the aqueous phase P1 in at least one first reactor R1 in the presence of a homogeneous or heterogeneous catalyst, obtaining a first aqueous fluid F1_1, comprising acrylic acid, at a pressure Π1;
- feeding this fluid F1_1 into another reactor R2;
- heating the fluid F1_1 introduced into reactor R2 in the presence of a catalyst at a pressure Π2, obtaining a fluid F1_2,
wherein Π2 and Π1 are different.

2. Process according to Claim 1, wherein phase P1 is obtained by a process comprising the process steps:
i) production of hydroxypropionic acid from a biological material, obtaining an aqueous phase comprising hydroxypropionic acid and microorganisms,
ii) optionally killing the microorganisms,
iii) separating solids from the aqueous phase.

3. Process according to one of the preceding claims, wherein phase P1 is aqueous and has a composition comprising
(Z1_1) 1 to 40 wt% of hydroxypropionic acid, salts thereof or mixtures thereof,
(Z1_2) 0.1 to 5 wt% of inorganic salts,
(Z1_3) 0.1 to 30 wt% of organic compounds other than hydroxypropionic acid,
(Z1_4) 0 to 50 wt% of solids, and
(Z1_5) 20 to 90 wt% of water,
wherein the sum of the components (Z1_1) to (Z1_5) is 100 wt%.

4. Process according to one of the preceding claims, wherein the purified phase has a purity of at least 40% with respect to acrylic acid.

5. Process according to one of the preceding claims, wherein the hydroxypropionic acid in phase P1 or the acrylic acid in phase P2 is transformed to its protonated form by adding acids, before carrying out at least one of the process steps (a2) or (a3) .

6. Process according to one of the preceding claims, wherein the hydroxypropionic acid is 2-hydroxypropionic acid.

7. Process according to one of Claims 1 to 5, wherein the hydroxypropionic acid is 3-hydroxypropionic acid.

8. Device for production of acrylic acid comprising the following units in fluid communication with one another:
- a synthesis unit for producing hydroxypropionic acid, followed by
- a dehydration step for converting the hydroxypropionic acid to acrylic acid, wherein the dehydration step has at least two reactors R1 and R2 in fluid communication with one another, of which one (R1) is loadable with a pressure Π1 and the other (R2) with a pressure Π2, the pressure Π2 being different from pressure Π1, followed by
- a unit for purifying the phase P2 comprising acrylic acid by suspension crystallization or layer crystallization, obtaining a purified phase, wherein reactor R2 is configured as a distillation or rectification device and reactor R1 is not.

9. Device according to Claim 8, wherein the purification unit comprises the following components in fluid communication with one another:
(δ1) a first crystallization zone, a second crystallization zone, a third crystallization zone, a first separation zone, a second separation zone, a third separation zone, at least one melter, and at least six lines,
(δ2) the first crystallization zone is connected to the first separation zone via a first line,
(δ3) the first separation zone is connected to the at least one melter via a second line,
(δ4) the at least one melter is connected to the second crystallization zone via a third line,
(δ5) the second crystallization zone is connected to the second separation zone via a fourth line,
(δ6) the second separation zone is connected to the third crystallization zone via a fifth line,
(δ7) the third crystallization zone is connected to the third separation zone via a sixth line.

10. Process for producing acrylic acid according to one of Claims 1 to 7, wherein a device according to Claim 8 or 9 is used.

## Revendications

1. Procédé de fabrication d'acide acrylique, comprenant les étapes de procédé suivantes :
(a1) la préparation d'une phase aqueuse P1 contenant de l'acide hydroxypropionique,
(a2) la déshydratation de l'acide hydroxypropionique pour obtenir une phase aqueuse P2 contenant de l'acide acrylique,
(a3) la purification de la phase aqueuse P2 contenant de l'acide acrylique, par cristallisation pour obtenir une phase purifiée,
dans lequel la déshydratation de l'acide hydroxypropionique à l'étape de procédé (a2) a lieu par déshydratation de la phase liquide par distillation réactive, dans lequel la déshydratation est réalisée dans au moins deux réacteurs, comprenant les étapes de procédé suivantes :
- le chauffage de la phase aqueuse P1 dans au moins un premier réacteur R1 en présence d'un catalyseur homogène ou hétérogène pour obtenir un premier fluide aqueux F1_1 contenant de l'acide acrylique à une pression π1 ;
- l'introduction de ce fluide F1_1 dans un autre réacteur R2 ;
- le chauffage du fluide F1_1 introduit dans le réacteur R2 en présence d'un catalyseur à une pression π2 pour obtenir un fluide F1_2 ;
π2 et π1 étant différentes.

2. Procédé selon la revendication 1, dans lequel la phase P1 est obtenue par un procédé comprenant les étapes de procédé suivantes :
i) la fabrication d'acide hydroxypropionique à partir d'un matériau biologique pour obtenir une phase aqueuse contenant de l'acide hydroxypropionique et des microorganismes,
ii) éventuellement l'élimination des microorganismes,
iii) la séparation des solides de la phase aqueuse.

3. Procédé selon l'une quelconque des revendications précédentes, dans lequel la phase P1 est aqueuse et présente une composition comprenant :
(Z1_1) 1 à 40 % en poids d'acide hydroxypropionique, ses sels ou leurs mélanges,
(Z1_2) 0,1 à 5 % en poids de sels inorganiques,
(Z1_3) 0,1 à 30 % en poids de composés organiques différents de l'acide hydroxypropionique,
(Z1_4) 0 à 50 % en poids de solides, et
(Z1_5) 20 à 90 % en poids d'eau,
la somme des composants (Z1_1) à (Z1_5) étant de 100 % en poids.

4. Procédé selon l'une quelconque des revendications précédentes, dans lequel la phase purifiée présente au regard de l'acide acrylique une pureté d'au moins 40 %.

5. Procédé selon l'une quelconque des revendications précédentes, dans lequel l'acide hydroxypropionique dans la phase P1 ou l'acide acrylique dans la phase P2 est transformé en sa forme protonée par ajout d'acides avant la réalisation d'au moins une des étapes de procédé (a2) ou (a3).

6. Procédé selon l'une quelconque des revendications précédentes, dans lequel l'acide hydroxypropionique est l'acide 2-hydroxypropionique.

7. Procédé selon l'une quelconque des revendications 1 à 5, dans lequel l'acide hydroxypropionique est l'acide 3-hydroxypropionique.

8. Dispositif pour la fabrication d'acide acrylique, contenant les unités suivantes, en communication fluidique les unes avec les autres :
- une unité de synthèse pour la fabrication d'acide hydroxypropionique, suivie par
- une étape de déshydratation pour la transformation de l'acide hydroxypropionique en acide acrylique, l'étape de déshydratation comprenant au moins deux réacteurs R1 et R2 en communication fluidique l'un avec l'autre, l'un d'entre eux (R1) étant résistant à une pression π1 et l'autre (R2) à une pression π2, la pression π2 étant différente de la pression π1, suivie par
- une unité de purification de la phase P2 contenant de l'acide acrylique par cristallisation en suspension ou cristallisation en couches pour obtenir une phase purifiée,
le réacteur R2 étant configuré sous la forme d'un dispositif de distillation ou de rectification, et le réacteur R1 ne l'étant pas.

9. Dispositif selon la revendication 8, dans lequel l'unité de purification comprend les constituants suivants, en communication fluidique les uns avec les autres :
(δ1) une première zone de cristallisation, une deuxième zone de cristallisation, une troisième zone de cristallisation, une première zone de séparation, une deuxième zone de séparation, une troisième zone de séparation, au moins un dispositif de fusion et au moins six conduites,
(δ2) la première zone de cristallisation est raccordée à la première zone de séparation par une première conduite,
(δ3) la première zone de séparation est raccordée audit au moins un dispositif de fusion par une deuxième conduite,
(δ4) ledit au moins un dispositif de fusion est raccordé à la deuxième zone de cristallisation par une troisième conduite,
(δ5) la deuxième zone de cristallisation est raccordée à la deuxième zone de séparation par une quatrième conduite,
(δ6) la deuxième zone de séparation est raccordée à la troisième zone de cristallisation par une cinquième conduite,
(δ7) la troisième zone de cristallisation est raccordée à la troisième zone de séparation par une sixième conduite.

10. Procédé de fabrication d'acide acrylique selon l'une quelconque des revendications 1 à 7, dans lequel un dispositif selon la revendication 8 ou 9 est utilisé.
